(19) Europäisches Patentamt
European Patent Office
Office européen des brevets

(11) EP 2 224 243 A1

(12) EUROPEAN PATENT APPLICATION

(43) Date of publication:
**01.09.2010 Bulletin 2010/35**

(51) Int Cl.:
***G01N 33/58*** *(2006.01)* ***G01N 33/542*** *(2006.01)*
***G01N 33/573*** *(2006.01)*

(21) Application number: **10152972.5**

(22) Date of filing: **08.09.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **12.09.2003 US 502377 P**

(62) Document number(s) of the earlier application(s) in accordance with Art. 76 EPC:
**04809691.1 / 1 671 128**

(71) Applicant: **Life Technologies Corporation**
**Carlsbad, CA 92008 (US)**

(72) Inventor: **Vogel, Kurt**
**Madison, WI WI 53717 (US)**

(74) Representative: **Lunt, Mark George Francis**
**Harrison Goddard Foote**
**Belgrave Hall**
**Belgrave Street**
**Leeds**
**LS2 8DD (GB)**

Remarks:
•This application was filed on 08-02-2010 as a divisional application to the application mentioned under INID code 62.
•Claims filed after the date of receipt of the application (Rule 68(4) EPC).

(54) **Identifying modulators of enzymatic activity**

(57) Compositions, including antibodies, polypeptides, and organic molecules, kits, apparatuses, and methods for probing molecular interactions using fluorescence polarization (FP) and/or time-resolved resonance energy transfer (TR-RET) are provided.

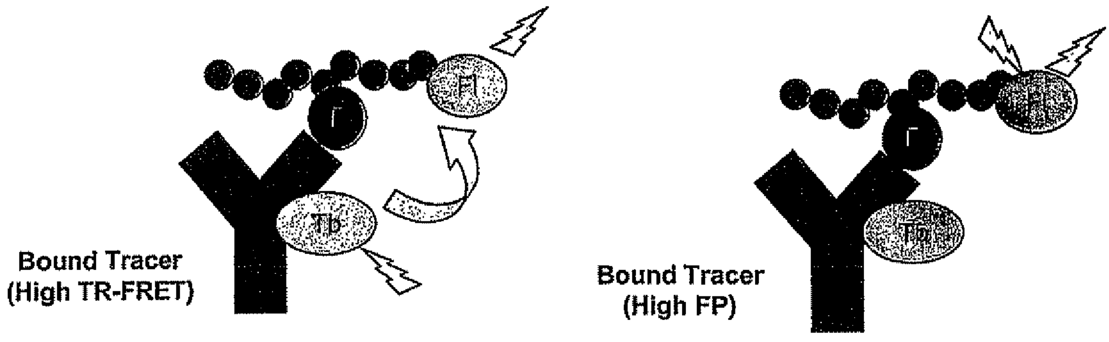


FIG. 1

## Description

### CROSS-REFERENCE TO RELATED APPLICATIONS

This application claims priority under 35 U.S.C. § 119(e) to U.S. Provisional Application Serial No. 60/502,377, filed on September 12,2003, which is incorporated by reference in its entirety herein.

### TECHNICAL FIELD

This invention relates to assays employing fluorescence polarization (FP) and/or time-resolved resonance energy transfer (TR-RET) detection methods, and more particularly to methods for monitoring and measuring molecular interactions, such as competitive binding or enzymatic activity events, using the same.

### BACKGROUND

Drug discovery can involve the systematic and/or high-throughput screening of diverse chemical libraries containing thousands of members. The size and complexity of these libraries, when coupled with the expense and length of the FDA approval process, have resulted in the need for simple, efficient, and homogeneous assays for probing molecular interactions.

Luminescence-based techniques, including fluorescence polarization (FP), resonance energy transfer (RET), and luminescence resonance energy transfer methods (LRET) methods, are typically highly sensitive, homogenous methods for probing molecular interactions. Background luminescence (e.g., fluorescence or luminescence from assay components), non-specific interactions of assay components, and light scattering, however, can limit the sensitivity of luminescence-based assays, particularly when luminophores having short lifetimes are used, resulting in the detection of false positives or false negatives in a drug screen. Follow-up screening of individually-picked compounds or the use of multiple screens may be required to validate screen results. It would be useful to have screening methodologies that could increase the information content of fluorescent or luminescent assays and reduce the number of spurious results encountered in drug screens.

### SUMMARY

The invention provides compositions, methods, apparatuses, and kits useful for monitoring molecular interactions, including competitive binding events and enzymatic activities. Accordingly, in one embodiment, the invention provides a method for measuring the effect of a test compound on binding between a first binding partner and a second binding partner. The method includes contacting a first binding partner, a second binding partner, and a test compound to form a test sample. In some embodiments, one of the first binding partner and the second binding partner includes a luminescent metal complex, while the other includes a fluorescent acceptor moiety. In other embodiments, one of the first binding partner and the test compound includes a luminescent metal complex, while the other includes a fluorescent acceptor moiety. A first binding partner and a second binding partner are capable of binding to one another to form a complex.

In the method, a test sample is exposed to polarized light and the polarization of fluorescent emission from the test sample is measured. The test sample can also be exposed to light having a wavelength in the range from 250 nm to 750 nm and the fluorescence emission of the test sample can be measured. The exposure to polarized light and the measurement of polarization of fluorescent emission can be performed prior to, simultaneously with, or after the exposure to light having a wavelength in the range from 250 nm to 750 nm and the measurement of fluorescence emission. The test compound is identified as affecting binding between the first binding partner and the second binding partner when the fluorescence polarization measurement or the fluorescence emission measurement, or both, of the test sample is different from the fluorescence polarization measurement or the fluorescence emission measurement of a corresponding control sample lacking the test compound.

A first binding partner and a second binding partner can be independently selected from the group consisting of a polypeptide, a polynucleotide, a lipid, a polysaccharide, a hormone, and a small organic compound. In some embodiments, a polypeptide can be an antibody or antibody fragment. A fluorescent acceptor moiety can be selected from the group consisting of fluorescein, rhodamine, GFP, GFP derivatives, FITC, 5-FAM, 6-FAM, 7-hydroxycoumarin-3-carboxamide, 6-chloro-7-hydroxycoumarin-3-carboxamide, fluorescein-5-isothiocyanate, dichlorotriazinylaminofluorescein, tetramethylrhodamine-5-isothiocyanate, tetramethylrhodamine-6-isothiocyanate, succinimidyl ester of 5-carboxyfluorescein, succinimidyl ester of 6-carboxyfluorescein, 5-carboxytetramethylrhodamine, 6-carboxymethylrhodamine, and 7-amino-4-methylcoumarin-3-acetic acid.

A luminescent metal complex can be a lanthanide metal complex. A lanthanide metal complex can include an

organic antenna moiety, a metal liganding moiety and a lanthanide metal ion. A lanthanide metal ion can be selected from the group consisting of Sm(III), Ru(III), Eu (III), Gd(III), Tb(III), and Dy(III). An organic antenna moiety can be selected from the group consisting of: rhodamine 560, fluorescein 575, fluorescein 590, 2-quinolone, 4-quinolone, 4-trifluoromethylcoumarin (TFC), 7-diethyl-amino-coumarin-3-carbohydrazide, 7-amino-4-methyl-2-coumaim (carbostyril 124), 7-amino-4-methyl-2-coumarin (coumarin 120), 7-amino-4-trifluoromethyl-2-coumarin (coumarin 124), and aminomethyltrimethylpsoralen. A metal liganding moiety can be a metal chelating moiety selected from the group consisting of: EDTA, DTPA, TTHA, DOTA, NTA, HDTA, DTPP, EDTP, HDTP, NTP, DOTP, DO3A, DOTAGA, and NOTA.

In some embodiments, a lanthanide metal complex has a structure:

$$-L_n\text{-}A\text{-}S_n\text{-}C_M,$$

or

$-L_n\text{-}C_M\text{-}S_n\text{-}A$, where A represents an organic antenna moiety; L represents a linker; S represents a spacer; n can be 0 or 1; C represents a metal chelating moiety; and M represents a lanthanide metal ion coordinated to C.

In another aspect, the invention provides a method for identifying a modulator of an enzymatic activity The method includes contacting an enzyme with a substrate for the enzyme, where the contacting is carried out under conditions effective for an enzymatic activity of the enzyme to form a product from the substrate and where the contacting is carried out in the presence of a potential modulator of the enzymatic activity The enzyme, substrate, and potential modulator are then contacted with a first binding partner and a tracer to form a test sample. The first binding partner has binding specificity for either the product or the substrate of the enzymatic activity. The first binding partner is capable of binding the tracer.

The tracer can be unlabeled or it can include a luminescent metal complex or a fluorescent acceptor moiety, e.g., a "luminescent tracer." For example, in one embodiment of the method, one of a first binding partner and a tracer includes a luminescent metal complex, while the other includes a fluorescent acceptor moiety. In other embodiments, one of a first binding partner and a substrate includes a luminescent metal complex, while the other includes a fluorescent acceptor moiety.

A test sample is then exposed to polarized light and the polarization of fluorescent emission from the test sample is measured. The test sample can also be exposed to light having a wavelength in the range from 250 nm to 750 nm and the fluorescence emission from the test sample is measured. The exposure to polarized light and the measurement of polarization of fluorescent emission can be performed prior to, simultaneously with, or after the exposure to light having a wavelength in the range from 250 nm to 750 nm and the measurement of fluorescence emission.

A potential modulator is identified as a modulator of the enzymatic activity when the fluorescence polarization measurement or the fluorescence emission measurement, or both, of the test sample is different from the fluorescence polarization measurement or fluorescence emission measurement, respectively, of a corresponding control sample lacking the potential modulator. In any of the methods described herein, a difference in the fluorescence polarization measurement of a test sample as compared to a control sample can be from about 30 mP to about 450 mP. A difference in fluorescence polarization measurement of a test sample as compared to a control sample can also be from about a 10% to about a 10,000% increase or decrease. The fluorescence emission of a test sample or a control sample can be measured at two or more wavelengths. A ratio of fluorescence emission measurements of a test sample or a control sample at two wavelengths can also be calculated.

An enzymatic activity can be selected from the group consisting of kinase activity, phosphatase, activity, glucuronidase activity, prenylation, glycosylation, methylation, demethylation, acylation, acetylation, ubiquitination, sulfation, proteolysis, nuclease activity, nucleic acid polymerase activity, nucleic acid reverse transcriptase activity, nucleotidyl transferase activity, and polynucleotide translation activity.

The invention also provides articles of manufacture. An article of manufacture, such as a kit, can include packaging material; and a first binding partner and/or a second binding partner, where the second binding partner is capable of binding the first binding partner. A binding partner can comprise a luminescent metal complex or a Fluorescent acceptor moiety.

In another aspect, the invention provides apparatuses. An apparatus can include a sample chamber; means for generating plane polarized light to illuminate the sample chamber; means for detecting polarized light emitted from the sample chamber; means for illuminating the sample chamber with light having a wavelength from 250 nm to 750 nm; and means for detecting light emitted from the sample chamber. In the apparatus, means for generating plane polarized light to illuminate the sample chamber and means for illuminating the sample chamber with light having a wavelength from 250 nm to 750 nm can be arranged so that both of the illumination means simultaneously illuminate the sample chamber with plane polarized light and light having a wavelength from 250 nm to 750 nm, respectively addition, means for detecting polarized light emitted from the sample chamber and means for detecting light emitted from the sample chamber can be arranged so that both detection means simultaneously detect the polarized light and the light emitted from said sample chamber.

In another aspect, the invention provides compositions. A composition can be a first binding partner, a second binding partner, or a mixture thereof. A binding partner can include a fluorescent acceptor moiety or a luminescent metal chelate.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting. All publications, patent applications, patents, and other references mentioned herein are incorporated by reference in their entirety. In case of conflict, the present specification, including definitions, will control.

The details of one or more embodiments of the invention are set forth in the accompanying drawings and the description below. Other features, objects, and advantages of the invention will be apparent from the description and drawings, and from the claims.

## DESCRIPTION OF DRAWINGS

FIG 1 is a schematic indicating one embodiment of a multiplex FP/TR-RET assay.

FIG 2 demonstrates the structure of a lanthanide metal chelate comprising an organic antenna moiety and the transfer of energy from the organic antenna moiety to the lanthanide metal ion.

FIG. 3 demonstrate the chemical structure of two luminescent metal chelates comprising organic antenna moieties.

FIG. 4 demonstrates the normalized excitation/emission spectrum for a terbium chelate comprising an organic antenna moiety (CS124).

FIG. 5 is a terbium chelate emission spectrum, demonstrating the overlap of terbium emission bands with fluorescein and rhodamine excitation bands and the location of fluorescein and rhodamine emission bands in regions having minimal terbium emission.

FIG. 6 demonstrates an overlap of the terbium chelate and fluorescein spectra.

FIG. 7 demonstrates an overlap of the terbium chelate and rhodamine spectra.

FIG. 8 demonstrates the relationship, at various fluorophore lifetimes, between expected polarization values (mP) and the MW of a complex between a first and second binding partner.

FIG. 9 demonstrates a direct binding assay using a Tb-chelate labeled antibody and a fluorescein-labeled phosphopeptide tracer.

FIG 10 demonstrates a competition assay measured with FP and TR-RET between a complex of a Tb-chelate labeled antibody and a fluorescein-labeled phosphopeptide tracer titrated with an unlabeled phosphopeptide competitor.

FIG. 11A and FIG. 11B demonstrate the results of screening a chemical library using both FP and TR- RET modes.

FIG. 12A and FIG. 12B demonstrate the results of measuring the interaction between an anti-phosphorylated CREB antibody and four different phosphorylated peptide tracers using FP and TR-RET measurements, respectively.

FIG. 13A and FIG. 13B demonstrate a titration of PKA enzyme measured by TR-RET and the Z'-factor for the assay, respectively.

FIG. 14A and FIG. 14B demonstrate comparable EC50 values obtained using FP and TR-RET to measure the interaction between Estrogen Receptor β and Estradiol.

FIG. 15 demonstrates the absorbance profile of a chelate and a chelate-antibody conjugate.

FIG. 16 demonstrates the polarization measurement versus antibody concentration at varying chelate:Ab ratios.

FIG. 17 demonstrates the normalized signal (FP or TR-RET) for a library screen.

FIG. 18 is a plot of normalized FP data vs. normalized TR-RET data for a library screen.

FIG. 19 is a plot of FP and TR-RET titration data obtained for two inhibitor compounds identified in a library screen.

FIG. 20 demonstrates that the TR-RET detection mode is resistant to background fluorescence signals.

FIG. 21 represents a re-analysis of spurious data obtained in a library screen, demonstrating the resistance of the TR-RET mode to light scattering.

FIG. 22 represents a re-analysis of spurious data obtained in a library screen.

FIG. 23A and 23B demonstrate the comparable EC50 values obtained using FP and TR-RET to measure the interaction between an antibody and a phosphopeptide.

FIG. 24A and 24B demonstrate the Z'-factors obtained in TR-RET mode and FP mode for the interaction of an antibody and a phosphopeptide.

FIG. 25 demonstrates FP and TR-RET data for a competition assay between a complex of an Eu-chelate labeled antibody and a labeled phosphopeptide tracer titrated with an unlabeled phosphopeptide competitor.

FIG. 26A and 26B demonstrate FP and TR-RET data obtained for a competition assay between a complex of a Tb-chelate-labeled anti-histag antibody and a labeled histag tracer titrated with an unlabeled histag-labeled protein competitor.

Like reference symbols in the various drawings indicate like elements.

## DETAILED DESCRIPTION

The invention is based on the discovery that the use of multiple detection modes (multiplex modes) can improve the sensitivity, reliability, and information content of assays that probe a wide variety of molecular interactions, including competitive binding events and enzymatic activities (e.g., post-translational modifications). Methods of the invention allow the use of both fluorescence polarization (FP) and time-resolved resonance energy transfer (TR-RET) detection modes. Use of the multiplex methods minimizes the number of false positive and false negative hits in screening assays, resulting in increased confidence in the integrity of screening results. In addition, use of the multiplex methods facilitates re-analysis of potentially spurious results due to background fluorescence interference and light scattering. Screening assays based on FP can be easily converted to TR-RET or multiplex FP/TR-RET assays using the methods of the present invention, allowing the probing of molecular interactions in either or both fluorescent modes. Compositions suitable for use in the presently described methods are also described, including mixtures of compositions.

### *Definitions*

Generally, the nomenclature used herein and many of the fluorescence, luminescence, computer, detection, chemistry, and laboratory procedures described herein are commonly employed in the art. Standard techniques are generally used for chemical synthesis, fluorescence or luminescence monitoring and detection, optics, molecular biology, and computer software and integration. Chemical reactions, cell assays, and enzymatic reactions are typically performed according to the manufacturer's specifications where appropriate. See, generally, Lakowicz, J.R. Topics in Fluorescence Spectroscopy, (3 volumes) New York: Plenum Press (1991), and Lakowicz, J. R. Emerging applications of florescence spectroscopy to cellular imaging: lifetime imaging, metal-ligand probes, multi photon excitation and light quenching, Scanning Microsc. Suppl. Vol. 10 (1996) pages 213-24, for fluorescence techniques; Sambrook et al., Molecular Cloning: A Laboratory Manual, 2ed. (1989) Cold Spring Harbor Laboratory Press, Cold Spring Harbor, N.Y., for molecular biology methods; Cells: A Laboratory Manual, 1st edition (1998) Cold Spring Harbor Laboratory Press, Cold Spring Harbor, N.Y., for cell biology methods; and *Optics Guide 5* Melles Griot® Irvine CA, and Optical Waveguide Theory, Snyder & Love (published by Chapman & Hall) for general optical methods, all of which are incorporated herein by reference.

General methods for performing a variety of fluorescent or luminescent assays on luminescent materials are known in the art and are described in, e.g., Lakowicz, J.R., Topics in Fluorescence Spectroscopy, volumes 1 to 3, New York: Plenum Press (1991); Herman, B., Resonance Energy Transfer Microscopy, in Fluorescence Microscopy of Living Cells in Culture, Part B, Methods in Cell Biology, vol. 30, ed. Taylor, D.L. & Wang, Y.-L., San Diego: Academic Press (1989), pp. 219-243; Turro, N.J., Modem Molecular Photochemistry, Menlo Park: Benjamin/Cummings Publishing Col, Inc. (1978), pp. 296-361; and Bernard Valeur, "Molecular Fluorescence: Principles and Applications" Wiley VCH, 2002. Guidance in the selection and use of specific resonance acceptor moieties is available at, for example, Berlman, I.B., Energy transfer parameters of aromatic compounds, Academic Press, New York and London (1973), which contains tables of spectral overlap integrals for the selection of resonance energy transfer pairs. Additional information sources include the Molecular Probes Catalog (2003) and website; and Tsien et al., 1990 Handbook of Biological Confocal Microscopy, pp. 169-178. Instruments useful for performing FP and/or RET and TR-RET applications are available from Tecan Group Ltd. (Switzerland) (Ultra, Ultra 384, Ultra Evolution); Perkin-Elmer (Boston, MA) (Fusion, EnVision, Victor V, and ViewLux), Amersham Bioscience (Piscataway, NJ) (LeadSeeker); and Molecular Devices Corporation (Sunnyvale, CA) (Analyst AD, GT, and HT).

Commonly used chemical abbreviations that are not explicitly defined in this disclosure may be found in The American Chemical Society Style Guide, Second Edition; American Chemical Society, Washington, DC (1997), "2001 Guidelines for Authors" J. Org. Chem. 66(1), 24A (2001), and "A Short Guide to Abbreviations and Their Use in Peptide Science" J. Peptide. Sci. 5, 465-471 (1999).

Abbreviations: t-Boc, tert-butyloxycarbonyl; Bzl, benzyl; PTK, protein tyrosine kinase; Fmoc, fluorenylmethyloxycarbonyl; ELISA, enzyme-linked immuno absorbant assay; FP, fluorescence polarization; FITC, fluorescein isothiocyanate; RET, resonance energy transfer; FRET, fluorescence resonance energy transfer or Forster resonance energy transfer; TR, time resolved; FAM, carboxyfluorescein.

As employed throughout the disclosure, the following terms, unless otherwise indicated, shall be understood to have the following meanings:

The terms "antibody" and "antibodies" include polyclonal antibodies, monoclonal antibodies, humanized or chimeric antibodies, single chain Fv antibody fragments, Fab fragments, and $F(ab)_2$ fragments. Polyclonal antibodies

are heterogeneous populations of antibody molecules that are specific for a particular antigen, while monoclonal antibodies are homogeneous populations of antibodies to a particular epitope contained within an antigen. A chimeric antibody is a molecule in which different portions are derived from different animal species, such as those having a variable region derived from a mouse monoclonal antibody and a human immunoglobulin constant region. The term "epitope" refers to an antigenic determinant on an antigen to which an antibody binds. Epitopes usually consist of chemically active surface groupings of molecules such as amino acids, sugar side chains, or chemical moieties (e.g., from organic compounds) and typically have specific three-dimensional structural characteristics as well as specific charge characteristics. Epitopes can consist of a series of contiguous amino acids, e.g., 5 contiguous amino acids. In other embodiments, an epitope can be a discontinuous epitope, e.g., the epitope is a particular arrangement of amino acids in space that results from the secondary, tertiary, and/or quaternary folding of a polypeptide. In yet other embodiments, an epitope can consist of a modified amino acid side chain, e.g., a phosphorylated tyrosine, serine, or threonine. Monoclonal antibodies are particularly useful in the present invention.

The term "RET" means resonance energy transfer, and refers to the radiationless transmission of an energy quantum from its site of absorption (the donor) to the site of its utilization (the acceptor) in a molecule, or system of molecules, by resonance interaction between donor and acceptor species, over distances considerably greater than interatomic, without substantial conversion to thermal energy, and without the donor and acceptor coming into kinetic collision. A donor is a moiety that initially absorbs energy (e.g., optical energy or electronic energy). A luminescent metal complex as described herein can comprise two donors: 1) an organic antenna moiety, which absorbs optical energy (e.g., from a photon); and 2) a lanthanide metal ion, which absorbs electronic energy (e.g., transferred from an organic antenna moiety). RET is sometimes referred to as fluorescent resonance energy transfer or Forster resonance energy transfer (both abbreviated FRET).

The term "acceptor" refers to a chemical or biological moiety that accepts energy via resonance energy transfer. In RET applications, acceptors may re-emait energy transferred from a donor fluorescent or luminescent moiety as fluorescence (e.g., RET or TR-RET) and are "fluorescent acceptor moieties." As used herein, such a donor fluorescent or luminescent moiety and an acceptor fluorescent moiety are referred to as a "RET pair." Examples of acceptors include coumarins and related fluorophores; xanthenes such as fluoresceins and fluorescein derivatives; fluorescent proteins such as GFP and GFP derivatives; rhodols, rhodamines, and derivatives thereof; resorufins; cyanines; difluoroboradiazaindacenes; and phthalocyanines. Acceptors, including fluorescent acceptor moieties, can also be useful as fluorescent probes in FP assays.

The terms "label" or "labeled" refer to the inclusion of a luminescent metal complex or a fluorescent acceptor moiety on a first binding partner, second binding partner, tracer, test compound, potential modulator, substrate, or product, as described herein.

The term "modulates" refers to partial or complete enhancement or inhibition of an activity or process (e.g., by attenuation of rate or efficiency).

The term "modulator" refers to a chemical compound (naturally occurring or non-naturally occurring), such as a biological macromolecule (e.g., polynucleotide, polypeptide, hormone, polysaccharide, lipid), an organic molecule (e.g., a small organic molecule), or an extract made from biological materials such as bacteria, plants, fungi, or animal (particularly mammalian, including human) cells or tissues. Modulators may be evaluated for potential activity as inhibitors or enhancers (directly or indirectly) of a biological process or processes (e.g., agonist, partial antagonist, partial agonist, inverse agonist, antagonist, antineoplastic agents, cytotoxic agents, inhibitors of neoplastic transformation or cell proliferation, cell proliferation-promoting agents, and the like) by inclusion in screening assays described herein. The activity of a modulator may be known, unknown, or partially known.

The term "non-naturally occurring" refers to the fact that an object, compound, or chemical cannot be found in nature. For example, a polypeptide or polynucleotide that is present in an organism (including viruses) that can be isolated from a source in nature and which has not been intentionally modified by man in the laboratory is naturally-occurring, while such a polypeptide or polynucleotide that has been intentionally modified by man is non-naturally occurring.

The term "organic molecule" refers to compounds having a molecular skeleton containing a covalent arrangement of one or more of the elements C, N, H, O, S, and P, and typically having a molecular weight less than 10000 Daltons. Organic molecules having a molecular weight less than 5000 Daltons may be referred to as "small organic molecules."

The term "polypeptide" refers to a polymer of two or more amino acids joined together through amide bonds. A polypeptide can be an entire protein (e.g., isolated from a natural source or an expression system), a fragment of a protein, an enzymatically or chemically synthesized and/or modified version of a protein or protein fragment, or an amino acid sequence designed *de novo* (e.g., not based on a known protein sequence). Polypeptides can be 2-1000 amino acids in length (e.g., 2-900, 2-800, 2-700, 2-600, 2-500, 2-480, 2-450, 2-300, 2-200, 2-100, 2-50, 2-25, 5-900, 5-800, 5-700, 5-600, 5-500, 5-450, 5-300, 5-200, 5-100, 5-50, 5-25, 10-900, 10-800, 10-700, 10-600, 10-500, 10-450, 10-300, 10-200, 10-100, 10-50, 20-900, 20-800, 20-700, 20-600, 20-500, 20-450, 20-300, 20-200, 20-100, or 20-50 amino acids

in length). Amino acids may be natural or unnatural amino acids, including, for example, beta-alanine, phenylglycine, and homoarginine. For a review, see Spatola, A.F., in Chemistry and Biochemistry of Amino Acids, Peptides and Proteins, B. Weinstein, eds., Marcel Decker, New York, p. 267 (1983). All of the amino acids used in the present invention may be either the D- or L-isomer. Particularly useful chemically modified or substituted amino acids including phosphorylated (e.g., phospho-serine (phosphorylated at the hydroxyl of the side chain), phospho-tyrosine (phosphorylated at the OH of the side-chain phenyl ring), and phospho-threonine (phosphorylated at the hydroxyl of the size chain)), sulfated, methylated, or prenylated amino acids.

The terms "post-translational modification" and "post-translational type modification" are used interchangeably and refer to enzymatic or neon-enzymatic modification of one or more amino acid residues in a polypeptide. Typical modifications include phosphorylation, dephosphorylation, glycosylation, methylation, sulfation, ubiquitination, acylation, acetylation, prenylation, and ADP-ribsoylation. Preferred post-translational type modifications include phosphorylation and dephosphorylation. The term post-translational modification includes non-covalent modifications that may affect polypeptide activity (e.g., protein activity), structure, or function, such as polypeptide-polypeptide interactions or the binding of ligands, allosteric modulators, other modulators, or second messengers such as calcium, cAMP, or inositol phosphates.

The term "test compound" refers to a compound to be tested by one or more screening method(s) of the invention, e.g., to determined if it is a putative modulator of an enzymatic activity such as a kinase activity. A test compound can be any chemical, such as an inorganic chemical, an organic molecule, a polypeptide, a carbohydrate, a polynucleotide, a polysaccharide, a lipid, a phospholipid, or a combination thereof. Typically, various predetermined concentrations (e.g., various dilutions) of test compounds are used for screening, such as 0.01 micromolar, 1 micromolar, or 10 micromolar. Experimental controls for a test compound can include measuring a signal for an assay performed in the absence of the test compound or comparing a signal obtained using a compound known to modulate a target activity with a signal obtained with the test compound.

### *Binding Partners*

The invention is based on monitoring and/or measuring a molecular interaction (e.g., complex formation or disruption) between two binding partners. A"binding partner" is a compound (e.g., a first binding partner) that has affinity for another compound (e.g., a second binding partner) (or vice versa) such that the two binding partners are capable of forming a complex when bound. Two binding partners can be members of a specific binding pair. For example, a first binding partner can be a monoclonal antibody and a second binding partner can be a composition having the epitope recognized by that monoclonal antibody.

Accordingly, in one aspect, the invention provides compositions that include a binding partner. The binding partner can be labeled with a luminescent metal complex. Alternatively, the binding partner can be labeled with a fluorescent acceptor moiety. Specific examples of binding partners labeled with luminescent metal complexes or fluorescent acceptor moieties are set forth in the Examples, below. The present invention also provides mixtures of binding partners. For example, a composition can include a first binding partner and a second binding partner. The first binding partner can comprise a luminescent metal complex while the second binding partner can comprise a fluorescent acceptor moiety. Alternatively, the first binding partner can comprise a fluorescent acceptor moiety., while the second binding partner can comprise a luminescent metal complex.

Typically, the affinity (apparent $K_d$) of a first binding partner for a second binding partner is about 1 mM or less, e.g., about 10 $\mu$M or less, or about 1 $\mu$M or less, or about 0.1 $\mu$M or less, or 10 nM or less, or 1 nM or less, or 0.1 nM or less. As one of skill in the art will recognize, one can systematically adjust experimental parameters, e.g., concentrations of assay components, reaction times, temperatures, and buffers, depending on the $K_d$ of the first binding partner for the second binding partner, to obtain a desired combination of conditions and cost-effectiveness.

A second binding partner need not be an optimal binding partner for a first binding partner. The term encompasses all binding partners whose binding interactions can be probed using the methods of the present invention. A second binding partner is sometimes referred to herein as a "tracer," and if it includes a luminescent metal complex or a fluorescent acceptor moiety, a "luminescent tracer."

A binding partner can be a polypeptide, a polynucleotide, a lipid, a phospholipid, a polysaccharide, or an organic molecule. Examples of specific polypeptide binding partners include an antibody, a protein, or an enzymatically or chemically-synthesized or modified polypeptide sequence (e.g., a polypeptide sequence derived from a protein, modified from a protein, or designed and synthesized *de novo*.) A polypeptide binding partner may be linear or cyclic. An organic molecule binding partner can be a small organic molecule.

Typical examples of first and second binding partners that form complexes include an antibody and a composition having an epitope or epitope mimetic recognized by that antibody; a polypeptide and a ligand (e.g., receptor-ligand interactions); a polypeptide and another polypeptide (e.g., protein-protein interactions); a polypeptide and a polynucleotide (e.g., protein-DNA or protein-RNA interactions); a polynucleotide and another polynucleotide (e.g., DNA-DNA,

DNA-RNA, or RNA-RNA interactions); a polypeptide and an organic molecule (e.g., protein-drug interactions); a polypeptide and a lipid (e.g., protein-phospholipid interactions); a polynucleotide and an organic molecule; and an organic molecule and another organic molecule.

A binding partner can comprise either a luminescent metal complex or a fluorescent acceptor moiety. In some embodiments of the methods described herein, one binding partner can comprise a luminescent metal complex and the other can comprise a fluorescent acceptor moiety, e.g., a first binding partner comprises a luminescent metal complex and a second binding partner comprises a fluorescent acceptor moiety. Inclusion of a luminescent metal complex and fluorescent acceptor moiety on a binding partner pair allows an interaction of first and second binding partners to be monitored by one or more fluorescent techniques (e.g., FP, TR-RET, or multiplex modes). For example, when a first binding partner and second binding partner are bound to one another, the complex will typically exhibit a characteristic FP or TR-RET signal (or bath). Disruption of the molecular interaction between the first binding partner and the second binding partner (e.g., by the addition of a competitor of the second binding partner) alters the FP or TR-RET signal (or both), allowing the monitoring of the molecular interaction in either FP or TR-RET modes (or both modes).

In one embodiment, an antibody can be labeled with a luminescent metal chelate and a polypeptide binding partner for the antibody can be labeled with a fluorescent acceptor moiety. When the antibody and polypeptide are bound to one another, the sample typically exhibits a high FP measurement and a fluorescence emission measurement characteristic ofRET between the luminescent metal chelate and the acceptor moiety. Addition of a competitor at a suitable concentration and with a suitable $K_d$ for the antibody results in displacement of the second binding partner, with a concomitant reduction in the FP measurement of the sample and a change in the fluorescence emission measurement as a result of a loss of RET between the luminescent metal chelate on the antibody and the fluorescent acceptor moiety on the polypeptide.

Binding partners can be prepared and purified by a number of methods known to those of ordinary skill in the art. For example, antibodies, including monoclonal antibodies and antibody fragments, can be prepared by a number of methods known to those of skill in the art, or can be purchased from a variety of commercial vendors, including Serotec (Raleigh, NC), Abcam (Cambridge, MA), R&D Systems, Cambridge Antibody Technologies, and Covance Research Products (Denver, CO).

In general, an antigen for which an antibody is desired is prepared, e.g., recombinantly, by chemical synthesis, or by purification of a native protein, and then used to immunize animals. For example, polypeptides containing a particular amino acid sequence and/or post-translational modification (e.g., phosphorylation) can be prepared by solid-phase chemical synthesis in order to raise an antibody specific for the sequence and/or post-translational modification. Various host animals including, for example, rabbits, chickens, mice, guinea pigs, goats, and rats, can be immunized by injection of the antigen of interest. Depending on the host species, adjuvants can be used to increase the immunological response and include Freund's adjuvant (complete and/or incomplete), mineral gels such as aluminum hydroxide, surface-active substances such as lysolecithin, pluronic polyols, polyanions, peptides, oil emulsions, keyhole limpet hemocyanin, and dinitrophenol. Polyclonal antibodies are contained in the sera of the immunized animals. Monoclonal antibodies can be prepared using standard hybridoma technology. In particular, monoclonal antibodies can be obtained by any technique that provides for the production of antibody molecules by continuous cell lines in culture as described, for example, by Kohler et al. (1975) Nature 256:495-497, the human B-cell hybridoma technique of Kosbor et al. (1983) Immunology Today 4:72, and Cote et al. (1983) Proc. Natl. Acad. Sci. USA 80:2026-2030, and the EBV-hybridoma technique of Cole et al., Monoclonal Antibodies and Cancer Therapy, Alan R. Liss, Inc. pp. 77-96 (1983). Such antibodies can be of any immunoglobulin class including IgM, IgG, IgE, IgA, IgD, and any subclass thereof The hybridoma producing the monoclonal antibodies of the invention can be cultivated *in vitro* or *in vivo.* Chimeric antibodies can be produced through standard techniques.

Antibody fragments that have specific binding affinity for an antigen can be generated by known techniques. Such antibody fragments include, but are not limited to, $F(ab')_2$ fragments that can be produced by pepsin digestion of an antibody molecule, and Fab fragments that can be generated by reducing the disulfide bridges of $F(ab')_2$ fragments. Alternatively, Fab expression libraries can be constructed. See, for example, Huse et al. (1989) Science 246:1275-1281. Single chain Fv antibody fragments are formed by linking the heavy and light chain fragments of the Fv region via an amino acid bridge (e.g., 15 to 18 amino acids), resulting in a single chain polypeptide. Single chain Fv antibody fragments can be produced through standard techniques, such as those disclosed in U.S. Patent No. 4,945,778.

Once produced, antibodies or fragments thereof can be tested for recognition of (and affinity for) a second binding partner by standard immunoassay methods including, for example, enzyme-linked immunosorbent assay (ELISA) or radioimmuno assay (RIA). See, Short Protocols in Molecular Biology, eds. Ausubel et al., Green Publishing Associates and John Wiley & Sons (1992). Suitable antibodies typically will have a $K_d$ for a second binding partner of about 1 mM or less, e.g., about 10 μM or less, or about 1 μM or less, or about 0.1 μM or less, or about 10 nM or less, or about 1 nM or less, or about 0.1 nM or less. For example, if a post-translationally modified protein is used to immunize an animal to produce an antibody specific for the particular post-translational modification, the second binding partner can be a polypeptide containing the same post-translational modification. In other embodiments, a second binding partner will

have the same chemical structure as an antigen used to immunize.

Other polypeptides in addition to antibodies are useful as first or second binding partners and can also be prepared and analyzed using standard methods. By way of example and not limitation, polypeptides can be obtained by extraction from a natural source (e.g., from isolated cells, tissues or bodily fluids), by expression of a recombinant nucleic acid encoding the polypeptide, or by chemical synthesis. Polypeptides can be produced by, for example, standard recombinant Technology, using expression vectors encoding the polypeptides. The resulting polypeptides then can be purified. Expression systems that can be used for small or large scale production of polypeptides include, without limitation, microorganisms such as bacteria (e.g., *E. coli* and *B. subtilis*) transformed with recombinant bacteriophage DNA, plasmid DNA, or cosmid DNA expression vectors; yeast (e.g., *S. cerevisiae*) transformed with recombinant yeast expression vectors; insect cell systems infected with recombinant virus expression vectors (e.g., baculovirus); plant cell systems infected with recombinant virus expression vectors (e.g., tobacco mosaic virus) or transformed with recombinant plasmid expression vectors (e.g., Ti plasmid); or mammalian cell systems (e.g., primary cells or immortalized cell lines such as COS cells, Chinese hamster ovary cells, HeLa cells, human embryonic kidney 293 cells, and 3T3 L1 cells) harboring recombinant expression constructs containing promoters derived from the genome of mammalian cells (e.g., the metallothionein promoter) or from mammalian viruses (e.g., the adenovirus late promoter and the cytomegalovirus promoter).

Suitable methods for purifying the polypeptides of the invention can include, for example, affinity chromatography, immunoprecipitation, size exclusion chromatography, and ion exchange chromatography. See, for example, Flohe et al. (1970) Biochim. Biophys. Acta. 220:469-476, or Tilgmann et al. (1990) FEBS 264:95-99. The extent of purification can be measured by any appropriate method, including but not limited to: column chromatography, polyacrylamide gel electrophoresis, or high-performance liquid chromatography.

Polypeptides as first or second binding partners can also be prepared using solid phase synthesis methods, see, e.g., WO 03/01115 and 6,410,255. For ease of synthesis and cost considerations, it is preferred that polypeptides synthesized chemically have between 3 to 50 amino acids (e.g., 3 to 30, 3 to 20, 3 to 15, 5 to 30, 5 to 20, 5 to 15, 8 to 20, 8 to 15, 10 to 10, 10 to 15 or 10 to 12 amino acids in length). In the polypeptides of the invention, a great variety of amino acids can be used. Suitable amino acids include natural, non-natural, and modified.(e.g., phosphorylated) amino acids. Amino acids with many different protecting groups appropriate for immediate use in the solid phase synthesis of peptides are commercially available.

Polynucleotides useful as binding partners can be produced by standard techniques, including, without limitation, common molecular cloning and chemical nucleic acid synthesis techniques. For example, polymerase chain reaction (PCR) techniques can be used. PCR refers to a procedure or technique in which target nucleic acids are enzymatically amplified. Sequence information from the ends of the region of interest or beyond typically is employed to design polynucleotide primers that are identical in sequence to opposite strands of the template to be amplified. PCR can be used to amplify specific sequences from DNA as well as RNA, including sequences from total genomic DNA or total cellular RNA. Primers are typically 14 to 40 nucleotides in length, but can range from 10 nucleotides to hundreds of nucleotides in length. General PCR techniques are described, for example in PCR Primer: A Laboratory Manual, ed. by Dieffenbach and Dveksler, Cold Spring Harbor Laboratory Press, 1995. When using RNA as a source of template, reverse transcriptase can be used to synthesize complementary DNA (cDNA) strands. Ligase chain reaction, strand displacement amplification, self-sustained sequence replication, or nucleic acid sequence-based amplification also can be used to obtain isolated nucleic acids. See, for example, Lewis Genetic Engineering News, 12(9):1 (1992); Guatelli et al., Proc. Natl. Acad. Sci. USA, 87:1874-1878 (1990); and Weiss, Science, 254:1292 (1991).

Polynucleotides of the invention also can be chemically synthesized, either as a single nucleic acid molecule (e.g., using automated DNA synthesis in the 3' to 5' direction using phosphoramidite technology) or as a series of smaller polynucleotides. For example, one or more pairs of long polynucleotides (e.g., >100 nucleotides) can be synthesized that contain the desired sequence, with each pair containing a short segment of complementarity (e.g., about 15 nucleotides) such that a duplex is formed when the polynucleotide pair is annealed. DNA polymerase is used to extend the polynucleotides, resulting in a single, double-stranded polynucleotide.

Polynucleotides of the invention also can be obtained by mutagenesis. For example, polynucleotide can be mutated using standard techniques including polynucleotide-directed mutagenesis and site-directed mutagenesis through PCR. See Short Protocols in Molecular Biology, Chapter 8, Green Publishing Associates and John Wiley & Sons, edited by Ausubel et al., 1992.

### *Luminescent Metal Complex*

A binding partner can comprise a luminescent metal complex. A luminescent metal complex can act as a donor fluorophore in a RET or TR-RET assay. A luminescent metal complex is useful in the present methods because its excited state lifetime is typically on the order of milliseconds or hundreds of microseconds rather than nanoseconds; a long excited state lifetime allows detection of a molecular interaction between binding partners to be monitored after the

decay of background fluorescence and/or interference from light-scattering.

Methods for covalently linking a luminescent metal complex to a variety of binding partners are known to those of skill in the art, see, e.g., WO 96/23526;WO 01/09188, WO 01/08712, and WO 03/011115; and U.S. Pat. Nos. 5,639,615; 5,656,433; 5,622,821; 5,571,897; 5,534,622; 5,220,012; 5,162,508; and 4,927,923.

A luminescent metal complex includes a metal liganding moiety, one or more lanthanide metal ions, and optionally linkers, spacers, and organic antenna moieties.

***Metal Liganding Moiety***

A metal liganding moiety coordinates one or more lanthanide metal ions to form a metal complex. Typically, a metal liganding moiety includes one or more metal coordinating moieties X, where X is a heteroatom electron-donating group capable of coordinating a metal cation, such as $O^-$, OH, $NH_2$, $OPO_3^{2-}$, NHR, or OR where R is an aliphatic group.

A metal liganding moiety can be a chelating moiety or a cryptand moiety. If a lanthanide metal ion is coordinated to a chelating moiety, the complex is referred to as a "metal chelate." If a lanthanide metal ion is coordinated to a cryptand moiety, the complex is referred to as a "metal cryptand."

A metal chelate should be stable to exchange of the lanthanide ion. Metal chelates preferably have a formation constant ($K_f$) of greater than $10^{10}$ $M^{-1}$. A variety of useful chelating moieties are known to those of skill in the art. Typical examples of cheating moieties include: EDTA, DTPA, TTHA, DOTA, NTA, HDTA, DTPP, EDTP, HDTP, NTP, DOTP, DO3A, DOTAGA, and NOTA.

In some embodiments, a luminescent metal chelate can have the following structures:

$$-L_n-A-S_n-C_M,$$

or

$$-L_n-C_M-S_n-A,$$

wherein A represents an organic antenna moiety;
L represents a linker;
S represents a spacer;
n can be 0 or 1;
C represents a metal cheating moiety; and
M represents a lanthanide metal ion coordinated to C.

For illustrative examples of luminescent metal chelates, see FIGs. 2 and 3. FIG. 3 also demonstrates luminescent metal chelates useful for conjugating to amine moieties (top structure) or thiol moieties (bottom structure) on binding partners.

Cryptates are formed by the inclusion of a lanthanide cation into a tridimensional organic cavity, leading to highly stable complexes. A variety of useful cryptand moieties are known to those of skill in the art. Examples of cryptand moieties useful in the present methods include: trisbypyridine (TBP, e.g, TBP pentacarboxylate), and pyridine bipyridine (e.g., pyridine bipyridine tetracarboxylate).

Chelating and cryptand moieties can be synthesized by a variety of methods known to those of skill in the art or may be purchased commercially. See U.S. Pat. Nos. 5,639,615; 5,656,433; 5,622,821; 5,571,897; 5,534,622; 5,220,012; 5,162,508; and 4,927,923; and WO 96/23526 and WO 03/011115.

***Lanthanide Metal Ions***

Metal liganding moieties coordinate one or more lanthanide metal ions to form a metal complex. Lanthanide metal ions are useful because their special electronic configuration shields the optically active electrons, resulting in characteristic line type emissions. As the electronic transitions of the metal ions are forbidden by quantum mechanics rules, the emission lifetimes of these ions are typically long (from μs to msec).

Useful lanthanide metal ions include Sm(III), Ru(III), Eu (III), Gd(III), Tb(III), and Dy(III). Methods for complexing a metal ion to a chelating or cryptand moiety are known to those of skill in the art, see, e.g., WO 96/23526 and WO 03/011115.

***Organic Antenna Moiety***

A luminescent metal complex can optionally include an organic antenna moiety. An organic antenna moiety

typically has a conjugated electronic structure so that it can absorb light. The absorbed light is transferred by intramolecular non-radiative processes from the singlet to the triplet excited state of the antenna moiety, then from the triplet state to the emissive level of the lanthanide ion, which then emits characteristically long-lived luminescence. See FIGs. 2 and 4. It should be noted that some metal liganding moieties can absorb light without the inclusion of an organic antenna moiety. For example, certain cryptand moieties that contain conjugated organic moieties, such as tribipyridine pentacarboxylate, do not require the inclusion of a discrete organic antenna moiety.

In some embodiments, an organic antenna moiety can be a polynuclear heterocyclic aromatic compound. The polynuclear heterocylic aromatic compound can have two or more fused ring structures. Examples of useful organic antenna moieties include rhodamine 560, fluorescein 575, fluorescein 590, 2-quinolone, 4-quinolone, 4-trifluoromethylcoumarin (TFC), 7-diethyl-amino-coumarin-3-carbohydrazide, 7-amino-4-methyl-2-coumann (carbostyril 124, CS 124), 7-amino-4-methyl-2-coumarin (coumarin 120), 7-amino-4-trifluoromethyl-2-coumarin (coumarin 124), and ammomethyltrimethylpsoralen. See FIGs. 2 and 3.

Compounds useful as organic antenna moieties can be synthesized by methods known to those of skill in the art or purchased commercially. See U.S. Pat. Nos. 5,639,615; 5,656,433; 5,622,821; 5,571,897; 5,534,622; 5,220,012; 5,162,508; and 4,927,923.

***Linkers, Spacers***

Linkers and Spacers can optionally be included in a luminescent metal complex. A Linker (L) functions to link a luminescent metal complex to a first or second binding partner. In some embodiments, a L can link an acetate, amine, amide, carboxylate, or methylene functionality on a metal liganding moiety to a first or second binding partner.

One of skill in the art can design Ls to react with a number of functionalities on binding partners, including, without limitation, amines, acetates, thiols, alcohols, ethers, esters, ketones, and carboxylates. In embodiments where the binding partner is a polypeptide, a L can cap the N-terminus, the C-terminus, or both N- and C- termini, as an amide moiety. Other exemplary L capping moieties include sulfonamides, ureas, thioureas and carbamates. Ls can also include linear, branched, or cyclic alkanes, alkenes, or alkynes, and phosphodiester moieties. The L may be substituted with one or more functional groups, including ketone, ester, amide, ether, carbonate, sulfonamide, or carbamate functionalities. Specific Ls contemplated also include NH--CO-NH-; $-CO-(CH_2)_n-NH-$, where n=1 to 10; -NH-Ph-; $-NH-(CH_2)_n-$, where n= 1 to 10; -CO-NH-; - $(CH_2)_n-NH-$, where n= 1 to 10; $-CO-(CH_2)_n-NH-$, where n=1 to 10; and -CS-NH-. Additional examples of Ls and synthetic methodologies for incorporating them into metal complexes, particularly metal complexes linked to polypeptides, are set forth in WO 01/09188, WO 01/08712, and WO 03/011115.

A Spacer (S) can connect an organic antenna moiety to a metal liganding moiety. In some embodiments, a S can link an acetate, amine, or methylene functionality on a metal liganding moiety to an organic antenna moiety. One of skill in the art can design Ss to react with a number of functionalities on organic antenna moieties and on metal liganding moieties, including, without limitation, amines, acetates, thiols, alcohols, ethers, esters, ketones, and carboxylates. Ss can include linear, branched, or cyclic alkanes, alkenes, or alkynes, and phosphodiester moieties. The S may be substituted with one or more functional groups, including ketone, ester, amide, ether, carbonate, sulfonamide, or carbamate functionalities. Specific Ss contemplated also include NH--CO-NH-; $-CO-(CH_2)_n-NH-$, where n= 1 to 10; -NH-Ph-; $-NH-(CH_2)_n-$, where n= 1 to 10; -CO-NH-;$-(CH_2)_n-NH-$, where n= 1 to 10; $-CO-(CH_2)_n-NH-$, where n=1 to 10; and -CS-NH-.

***Fluorescent Acceptor Moiety***

A binding partner can include a fluorescent acceptor moiety. A fluorescent acceptor moiety can act as an acceptor in RET or TR-RET-based assays and/or can be a fluorophore for which the polarization of fluorescence emission is measured in an FP-based assay.

In general, a fluorescent acceptor moiety should exhibit a good quantum yield and a large extinction coefficient; should be resistant to collisional quenching and bleaching; and should be easily conjugated to a variety of first and second binding partners by methods known to those having ordinary skill in the art. Suitable fluorophores include, without limitation, fluorescein, rhodamine, FITCs (e.g., fluorescein-5-isothiocyanate), 5-FAM, 6-FAM, 5,6-FAM, 7-hydroxycoumarin-3-carboxamide, 6-chloro-7-hydroxycoumarin-3-carboxamide, dichlorotriazinylaminofluorescein, tetramethylrhodamine-5-isothiocyanate, tetramethylrhodamine-6-isothiocyanate, succinimidyl ester of 5-carboxyfluorcscein, succinimidyl ester of 6-carboxyfluorescein, 5-carboxytetramethylrhodamine, 6-carboxymethylrhodamine, and 7-amino-4-methylcoumarin-3-acetic acid. Other suitable fluorophores include the Cy family of fluorophores (Cy 3, Cy3B, Cy3.5, Cy5; available from Amersham Biosciences, Piscataway, NJ); the Alexa Fluor family (available from Molecular Probes, Eugene, OR); the BODIPY family (available from Molecular Probes, Eugene, OR); carbopyronins; squarines; cyanme/indocyanines; benzopyrylium heterocyles; and amide-bridged benzopyryliums.

Fluorescent proteins and mutants can also be used as fluorescent acceptor moieties. Examples include firefly,

bacterial, or click beetle luciferases, aequorins, and other photoproteins (for example as described in U.S. Patents 5,221,623, issued June 22, 1989 to Thompson et al., 5,683,888 issued November 4, 1997 to Campbell; 5,674,713 issued September 7, 1997 to DeLuca et al.; 5,650,289 issued July 22, 1997 to Wood; and 5,843,746 issued December 1, 1998 to Tatsumi et al.). GFP and GFP mutants are particularly useful in applications using Tb(III)-containing metal complexes. A variety of mutants of GFP from *Aequorea victoria* have been created that have distinct spectral properties, improved brightness, and enhanced expression and folding in mammalian cells compared to the native GFP (e.g., see Table 7 of U.S. 6,410,255 and also Green Fluorescent Proteins, Chapter 2, pages 19 to 47, edited by Sullivan and Kay, Academic Press; U.S. patent Nos: 5,625,048 to Tsien et al., issued April 29, 1997; 5,777,079 to Tsien et al., issued July 7, 1998; and U.S. Patent No. 5,804,387 to Cormack et al., issued September 8, 1998).

A fluorescent acceptor moiety for use in multiplex assays should exhibit characteristics useful for both RET/TR-RET applications and FP applications. For example, for FP assays, a fluorophore preferably exhibits a fluorescent excited state lifetime of at least 1 nanosecond, or at least 2 nanoseconds. For TR-RET applications, a region of the fluorophore's absorbance spectra should overlap with a region of a luminescent metal chelate's emission spectra, while a region of the fluorophore's emission spectra should not overlap substantially with a region of the luminescent metal chelate's emission spectra. For example, FIG 5 demonstrates overlap of the emission spectra of an organic antenna-Tb(III)-chelate-containing metal complex with the absorption spectra of fluorescein and rhodamine. FIG 5 also demonstrates that regions of the emission spectra of the organic-antenna-Tb(II)-chelate-containing metal complex do not overlap with the emission spectra for fluorescein and rhodamine. See also FIGs. 6-7.

Examples of suitable acceptor fluorophores in TR-RET assays using Tb(III)-containing luminescent metal complexes include: fluorescein (and its derivatives); rhodamine (and its derivatives); Alexa Fluors 488, 500,514,532, 546, 555, 568 (available from Molecular Probes); BODIPYs FL, R6G, and TMR (available from Molecular Probes); Cy3 and Cy3B (available from Amersham Biosciences), and IC3 (available from Dojindo Molecular Technologies, Gaithersburg, MD). Examples of suitable acceptor fluorophore in TR-RET assays using Eu(III)-containg luminescent metal complexes include: Alexa Fluors 594, 610, 633, 647, and 660 (available from Molecular Probes); BODIPYs TR, 630/650, and 650/665 (available from Molecular Probes); Cy5 (available from Amersham Biosciences) and IC5 (available from Dojindo Molecular Technologies).

Suitable fluorophores for use in the present invention are commercially available, e.g., from Molecular Probes (Eugene, OR), Attotec (Germany), Amersham, and Biosearch Technologies (Novato, CA). Methods for incorporating fluorophores into a variety of binding partners are known to those of skill in the art; see, e.g., 6,410,255.

### *Methods and Assays*

Methods of the present invention are based on the finding that luminescent or fluorescent assays based on FP or RET (including TR-RET) can be easily converted into multiplex assays, allowing detection in multiple luminescent modes. For example, assays based on measuring a change in FP of one binding partner can be easily converted to TR-RET assays by the incorporation of an appropriate luminescent metal complex on one binding partner and a fluorescent acceptor moiety on the other binding partner. In some embodiments, a binding partner that includes a fluorescent acceptor moiety useful for FP measurements need not be modified, provided that a suitable luminescent metal complex is chosen for inclusion on the other binding partner, as described above. Any of the methods described herein can be homogeneous or heterogeneous.

### *Fluorescence Polarization*

Methods of the present invention take advantage of a change in fluorescence polarization upon first and second binding partner complex formation or disruption. Polarization measurements are based on the relative rotational movement of a fluorophore compared to the excited state life-time of that fluorophore. For globular molecules in dilute solution, the relationship between polarization (p) and the degree of rotational movement can be readily derived (see Weber, Polarization of the fluorescence of solutions, in Fluorescence and Phosphorescence Analysis, Don Hercules (ed.), Interscience Publishers, New York, Chapter 8, pages 217-240 (1966)). Rotational movement can be related to the rotational diffusion constant of the molecule, and hence to the molecular volume. In practice there is often a close correlation between molecular size and relative polarization of emitted light from a fluorophore, although effects due to conformational freedom of a molecule, even when bound, are hard to predict. See FIG. 8 for a graph of expected polarization values for different fluorophore lifetimes and MWs of complexes of a first and second binding partner.

A change in the fluorescence polarization of a sample can occur when a complex of a first and second binding partner (one of which comprises a fluorescent acceptor moiety) is either formed or disrupted. Complex formation or disruption can result in from about a 10% to about a 10,000% increase or decrease in the fluorescence polarization measurement of a sample. Preferably, a change in the fluorescence polarization measurement is from about 30 mP to about 450 mP.

Polarization-based assays are relatively easy to set up and can be obtained over a wide concentration, temperature, and ionic strength range. See, e.g., U.S. 6,511,815 and 5,445,935.

***RET and TR-RET***

Methods of the present invention also take advantage of resonance energy transfer between a luminescent metal chelate and a fluorescent acceptor moiety (RET). A donor luminescent metal chelate is excited by light of appropriate wavelength and intensity (e.g., within the donor antenna moiety's excitation spectrum) and under conditions in which direct excitation of the acceptor fluorophore is minimized. The donor luminescent chelate then transfers the absorbed energy by non-radiative means to the acceptor fluorescent moiety, which subsequently re-emits some of the absorbed energy as fluorescence emission at one or more characteristic wavelengths. In TR-RET applications, the re-emitted radiation is not measured until after a suitable delay time, e.g., 25, 50,75,100,150,200, or 300 microseconds to allow decay of background fluorescence, light scattering, or other luminescence, such as that caused by the plastics used in microtiter plates.

In some RET applications, a first binding partner can comprise either a luminescent metal complex or a fluorescent acceptor moiety, while the second binding partner comprises the other. For example, an antibody first binding partner can be labeled with a Tb(III)-chelate-orgnic antenna moiety (luminescent metal chelate), while a polypeptide for which the antibody is specific can be labeled with a fluorescein (fluorescent acceptor moiety). In this case, disruption of the complex formed by the antibody and polypeptide (e.g., by a compound that affects binding between the two) results in an alteration in energy transfer between the luminescent metal chelate on the antibody and the fluorescent acceptor moiety on the polypeptide that may be used to monitor and measure the binding between the first and second binding partners. A compound that affects binding of a second binding partner (or tracer) to a first binding partner can be, for example, a test compound, an enzyme product (e.g., for which the first binding partner has specificity), or an enzyme substrate (e.g., for which the first binding partner has specificity).

In other RET embodiments, a compound that affects binding of a second binding partner (or tracer) to a first binding partner can comprise either a luminescent metal chelate or fluorescent acceptor moiety while the first binding partner comprises the other. In these embodiments, disruption of the complex formed between the first binding partner and the second binding partner by the labeled compound that affects binding can result in an increase in RET.

RET can be manifested as a reduction in the intensity of the luminescent signal from the donor luminescent metal complex and/or an increase in emission of fluorescence from the acceptor fluorescent moiety. For example, when a complex between an antibody having a donor luminescent metal complex and a polypeptide having an acceptor fluorescent moiety is disrupted, e.g., by a competitor for the polypeptide, such as an unlabeled polypeptide, the donor luminescent metal complex and the acceptor fluorescent moiety physically separate, and RET is diminished or eliminated. Under these circumstances, luminescence emission from the donor luminescent metal complex increases and fluorescence emission from the acceptor fluorescent moiety decreases. Accordingly, a ratio of emission amplitudes at wavelengths characteristic (e.g., the emission maximum) of the donor luminescent metal complex relative to the acceptor fluorescent moiety should increase as compared to the same ratio under RET conditions (e.g., when emission of the donor luminescent metal complex is quenched by the acceptor).

The efficiency of RET is dependent on the separation distance and the orientation of the donor luminescent metal complex and acceptor fluorescent moiety, the luminescent quantum yield of the donor metal ion, the spectral overlap with the acceptor fluorescent moiety, and the extinction coefficient of the acceptor fluorophore at the wavelengths that overlap with the donor's emission spectra. Forster derived the relationship:

$$E = (F^\circ - F)/F^\circ = Ro^6/(R^6 + Ro^6)$$

where E is the efficiency of RET, F and F° are the fluorescence intensities of the donor in the presence and absence of the acceptor, respectively, and R is the distance between the donor and the acceptor. Ro, the distance at which the energy transfer efficiency is 50% of maximum is given (in A) by:

$$Ro = 9.79 \times 10^3 (K^2 QJn^{-4})^{1/6}$$

where $K^2$ is an orientation factor having an average value close to 0.67 for freely mobile donors and acceptors, Q is the

quantum yield of the unquenched fluorescent donor, n is the refractive index of the intervening medium, and J is the overlap integral, which expresses in quantitative terms the degree of spectral overlap. The characteristic distance Ro at which RET is 50% efficient depends on the quantum yield of the donor, the extinction coefficient of the acceptor, the overlap between the donor's emission spectrum and the acceptor's excitation spectrum, and the orientation factor between the two fluorophores.

Changes in the degree of RET can be determined as a function of a change in a ratio of the amount of luminescence from the donor and acceptor moieties, a process referred to as "ratioing." By calculating a ratio, the assay is less sensitive to, for example, well-to-well fluctuations in substrate concentration, photobleaching and excitation intensity, thus making the assay more robust. This is of particular importance in automated screening applications where the quality of the data produced is important for its subsequent analysis and interpretation. See, e.g., U.S. 6, 410, 255; 4,822,733; 5,527,684; and 6,352,672.

For example, in some embodiments of the method, a ratiometric analysis is performed, wherein a ratio of luminescence emission at two different wavelengths is compared between a test sample and a control sample. In a typical TR-RET-based assay, the two wavelengths can correspond to an emission maximum for a luminescent metal complex and a fluorescent acceptor moiety. In some embodiments, an emissions ratio of the control sample will be about 1.5, 2, 3, 4, 5, 7, 10, 15, 20, 25, 30, 40, 50, or 100 times larger or smaller than the emissions ratio of a test sample.

***Methods for Measuring Effects of Test Compounds on Binding Between Binding Partners***

Methods of the present invention can be used to measure the effect of a test compound on binding between a first binding partner and a second binding partner. For example, the present methods may be used to identify competitive binders to first or second binding partners, or to identify compounds that physically (e.g., allosterically) or chemically affect a first or second binding partner so as to consequently affect binding of its partner. Accordingly, assays to identify effects of test compounds on such binding partner interactions as protein-protein interactions, protein-ligand interactions, protein-DNA interactions, and polynucleotide hybridizations may be designed using the present methods.

In one method, a first binding partner, a second binding partner, and a test compound are contacted to form a test sample. In some embodiments, one of the binding partners comprises a luminescent metal complex, while the other comprises a fluorescent acceptor moiety. See FIG 1. As described previously, the first and second binding partner are capable of binding to one another to form a complex. The test sample is exposed to polarized light at an appropriate wavelength (e.g., at a wavelength in an absorbance band of the fluorescent acceptor moiety) and the polarization of fluorescent emission from the test sample is measured. In some embodiments, the test sample is also exposed to light (e.g., at a wavelength in an absorbance band of the luminescent metal complex), typically in the wavelength range of 250 nm to 750 nm, and the fluorescence emission from the test sample is measured. Fluorescence emission may be measured after a suitable time delay, as indicated above, to result in a time-resolved fluorescence emission measurement.

In embodiments where a test sample is exposed to polarized light and also light (e.g., having a wavelength in the range from 250 nm to 750 nm), the exposure to polarized light and measurement of polarization may be performed before, after, or simultaneously with the exposure to light and measurement of fluorescence emission from the test sample. For example, the exposure to polarized light and measurement of polarization may be performed up to 5 sec., 10 sec., 20 sec., 30 sec., 1 min., 2 min., 5 min., 10 min., 1 hr., 5 hrs., or 24 hrs. before or after the exposure, to light and measurement of fluorescence emission from the test sample.

In other embodiments, as explained above, a test compound can comprise either a luminescent metal complex or a fluorescent acceptor moiety and a first binding partner can comprise the other. For example, a first binding partner receptor can be labeled with a luminescent metal chelate while a test ligand for the first binding partner receptor can be labeled with a fluorescent acceptor moiety. Disruption of a complex formed between the first binding partner receptor and an unlabeled second binding partner (e.g., a ligand for the receptor) by the labeled test ligand can lead to an increase in RET and/or FP.

A test compound is identified as affecting binding between first and second binding partners when the fluorescence polarization measurement or the fluorescence emission measurement of the test sample, or both, is different from the fluorescence polarization measurement or the fluorescence emission measurement of a control sample lacking the test compound. Generally, there should be a statistically significant difference in one or both measurements as compared to the control sample. As one of skill in the art will recognize, whether or not a difference is statistically significant will depend on the type of measurement and the experimental conditions. It is understood that when comparing measurements, a statistically significant difference indicates that the test compound may warrant further study. Typically, a difference is considered statistically significant at $p < 0.05$ with an appropriate parametric or non-parametric statistic, e.g., Chi-square test, Student's t-test, Mann-Whitney test, or F-test. In some embodiments, a difference is statistically significant at $p < 0.01$, $p < 0.005$, or $p < 0.001$.

***Methods for Identifying Modulators of Enzymatic Activity***

Methods of the invention can also be used to identify a modulator of enzymatic activity. In these embodiments, a first binding partner is selected based on specificity for either a substrate or a product of an enzymatic activity. For example, an antibody with specificity for a phosphorylated tyrosine as compared to an unmodified tyrosine can be a first binding partner with specificity for a product of tyrosine kinase activity. A tracer is then selected based partially on the specificity of the first binding partner for the substrate or product of the enzymatic activity. For example, a tracer can include the purported epitope recognized by an antibody first binding partner, or a recognition site or chemical structure recognized by a polypeptide first binding partner. In other embodiments, a tracer can have the same chemical structure as an antigen used to immunize an animal to generate a first binding partner antibody. Typically, the first binding partner will bind to a tracer with a similar $K_d$ as to the enzymatic product or substrate for which it has specificity, e.g., about 0.001 to 1000 times, or 0.01 to 100 times, or 0.1 to 10 times the $K_d$ of the first binding partner for the product or substrate.

A tracer may be labeled (e.g., include a luminescent metal complex or a fluorescent acceptor moiety; referred to herein as a "luminescent tracer") or the tracer may be unlabeled. For example, if the first binding partner is an antibody with specificity for a phosphorylated tyrosine, a product of tyrosine kinase activity, a luminescent tracer can be selected that includes the epitope (or an epitope mimetic) recognized by the antibody (in this case, a phosphorylated tyrosine) so that the antibody binds the luminescent tracer. The inclusion of a fluorescent acceptor moiety or luminescent metal complex on the tracer should not substantially affect the $K_d$ of the first binding partner for the tracer.

Because the assay is based on the selection of a first binding partner having specificity for a product or substrate of an enzymatic activity, a wide variety of enzymatic activities may be probed, including, without limitation, kinase activity, phosphatase activity, glucuronidase activity, prenylation, glycosylation, methylation, demethylation, acylation, acetylation, ubiquitination, sulfation, proteolysis, nuclease activity, nucleic acid polymerase activity, nucleic acid reverse transcriptase activity, nucleotidyl transferase activity, polynucleotide transcription activity, and polynucleotide translation activity.

In the method, an enzyme is contacted with a substrate for the enzyme under conditions effective for an enzymatic activity of the enzyme to form a product from the substrate. As one of skill in the art will recognize, conditions effective for enzymatic activity will vary with the enzyme, enzymatic activity, and substrate chosen. For kinase reactions, ATP is generally included. Incubation conditions for a contacting step can vary, e.g., in enzyme concentration, substrate concentration, temperature, and length of time. Incubation temperature conditions typically can be from about 15 to about 40 °C; in some embodiments, the temperature may be about room temperature, e.g., about 20-25 °C.

A contacting step is carried out in the presence of a potential modulator of the enzymatic activity. In some embodiments, the enzyme, substrate, and potential modulator mixture is then contacted with a first binding partner and luminescent tracer, as described above, to form a test sample. As indicated previously, in these embodiments, either the first binding partner or the luminescent tracer includes a luminescent metal complex, while the other includes a fluorescent acceptor moiety.

In other embodiments, the enzyme, substrate, and potential modulator mixture is contacted with a first binding partner and a tracer to form a test sample. In these embodiments, either the first binding partner or the substrate includes a luminescent metal complex, while the other includes a fluorescent acceptor moiety. In such cases, enzymatic activity can result in the conversion of the labeled substrate to a labeled product. The inclusion of a fluorescent acceptor moiety or luminescent metal complex on the substrate should not substantially affect the ability of the enzyme to form a product from the labeled substrate. In addition, the inclusion of a fluorescent acceptor moiety or luminescent metal complex on the substrate (or product) should not substantially affect the $K_d$ of the first binding partner for the substrate (or product) for which it has specificity.

The test sample is exposed to polarized light at an appropriate wavelength (e.g., at a wavelength in an absorbance band of the fluorescent acceptor moiety) and the polarization of fluorescent emission from the test sample is measured. In some embodiments, the test sample is also exposed to light (e.g., at a wavelength in an absorbance band of the luminescent metal complex), typically in the wavelength range of 250 nm to 750 nm, and the fluorescence emission from the test sample is measured. Fluorescence emission may be measured after a suitable time delay, as indicated above, to result in a time-resolved fluorescence emission measurement. As indicated previously, the exposure to polarized light and measurement of polarization may be performed before, after, or simultaneously with the exposure to light and measurement of fluorescence emission from the test sample.

As described above, in some embodiments a tracer maybe unlabeled, e.g., in embodiments where a first binding partner is labeled with a luminescent metal complex and a substrate is labeled with a fluorescent acceptor moiety. Disruption of a complex formed between an unlabeled tracer and a labeled first binding partner by an appropriately labeled compound (e.g., labeled substrate, labeled product, labeled test compound) that affects binding between the unlabeled tracer and first binding partner can lead to an increase or decrease in RET, FP, or both.

A potential modulator is identified as a modulator of enzymatic activity when the fluorescence polarization measurement or the fluorescence emission measurement of the test sample, or both, is different from the fluorescence

polarization measurement or the fluorescence emission measurement of a control sample lacking the potential modulator. As indicated above, there should be a statistically significant difference as compared to the control sample. As one of skill in the art will recognize, whether or not a difference is statistically significant will depend on the type of measurement and the experimental conditions. It is understood that when comparing measurements, a statistically significant difference indicates that that potential modulator may warrant further study. Typically, a difference is considered statistically significant at $p < 0.05$ with an appropriate parametric or non-parametric statistic, e.g., Chi-square test, Student's t-test, Mann-Whitney test, or F-test. In some embodiments, a difference is statistically significant at $p < 0.01$, $p < 0.005$, or $p < 0.001$.

Any of the methods of the present invention can be modified to be performed in a high-throughput or ultra-high-throughput manner. For example, a method to identify a modulator of activity of an enzyme may be modified to contact a plurality of substrates, independently, with a particular enzyme and potential modulator, to form a plurality of enzyme mixtures. Each enzyme mixture is then contacted with an appropriate first binding partner and luminescent tracer to form a test sample, with the excitation (e.g., exposure to plane polarized light and exposure to light) and measurement (of emission of polarized and fluorescent light) steps as described previously. As one of skill in the art will appreciate, such high-throughput methods are particularly amenable to multi-well plate or 2-D array panel formats. Devices for incubating and monitoring multi-well plates are known in the art.

The dynamic range, quality, and robustness of the methods of the present invention can be evaluated statistically. For example, the Z'-Factor is a statistic designed to reflect both assay signal dynamic range and the variation associated with signal measurements. Signal-to-noise (S/N) or signal-to-background (S/B) ratios alone are unsatisfactory in this regard because they do not take into account the variability in sample and background measurements and signal dynamic range. The Z'-Factor takes into account these factors, and because it is dimensionless, it can be used to compare similar assays. Typically, assays of the present invention yield Z'-factors of greater than or equal to 0.5. Methods for determining Z'-factor are known to those of skill in the art. A Z'-factor may be determined by evaluating the dynamic range of a method.

***Articles of Manufacture and Apparatuses***

The invention also provides articles of manufacture, such as kits, and apparatuses useful for performing the described inventions. Typically, a kit includes packaging material, such as a container, and one or more compositions useful as first and/or second binding partners. In some embodiments, a kit can include one or more of the following: a multi-well plate, one or more enzymes, buffers, and directions for use of the kit.

An apparatus will generally include a sample chamber, means for generating plane polarized light to illuminate the sample chamber; and means for illuminating the sample chamber with light having a wavelength from 250 nm to 750 nm. In addition, an apparatus will include means for detecting polarize light emitted from the sample chamber and means for detecting light (e.g., fluorescence) emitted from the sample chamber. In some embodiments, both illumination means illuminate the sample chamber simultaneously. In other embodiments, both detections means detect the polarized light and the light emitted (e.g., fluorescence) simultaneously.

**EXAMPLES**

Example 1 - Labeling of Antibody with a Luminescent Metal Chelate

1 mg purified PY72 (anti-phosphotyrosine) IgG antibody, an antibody that preferentially binds amino acid sequences containing phosphorylated tyrosines (e.g., sequences phosphorylated by protein tyrosine kinases (PTKs)) and was dialyzed for 1.5 hours in a 100 mM sodium bicarbonate buffer, pH 9.5, using a 12-14,000 MWCO dialysis membrane. [PY72 hybridoma cells were obtained from the Salk Institute; the immunogen was phosphotyrosine conjugated to KLH. Ascites were produced by Harlan Bioproducts for Science, Indianapolis IN. Ascites were purified with a protein G column (Pierce). Purified antibody is also available from Covance, Berkeley CA (Part # MMS414P).] The antibody was then removed from the dialysis membrane and concentrated to 48.8 uM (7.3 mg/mL) using a Centricon YM50 (Millipore) concentrator. 100 uL of this antibody solution was diluted to 5 mg/ml (33.4 uM) into the labeling reaction which consisted of 10 mM phenyl phosphate, and 660 $\mu$M carbostyril 124-diethylenetriaminepentaaceticacid-phenylalanine isothiocyanate *Tb(III) (CS124-DTPA-Phe-NCS*Tb, see FIG. 3) (final concentrations) in 100 mM sodium bicarbonate buffer, pH 9.5. The reaction was incubated at room temperature for 4 hours with light vortexing every 30 minutes, and then dialyzed twice for 1.5 hours each against tris-buffered saline (TBS) to remove unreacted and/or hydrolyzed chelate. The amount of chelate bound to the antibody was quantitated by the absorbance of the CS124 moiety at 343 nm ($E_{340}$ = 11,440 $M^{-1}cm^{-1}$), and the amount of antibody quantitated by its absorbance at 280 nm ($E_{280}$ = 210,000 $M^{-1}cm^{-1}$), correcting for the absorbance of the CS124 at 280 nM (1.1 times its absorbance at 343 nM). From these measurements it was determined that the reaction produced an antibody labeled with an average of 5.8 chelates per antibody.

A monoclonal antibody with specificity for phosphorylated serines (anti-pSer; phosphorylated serines are products of Serine/Threonine kinase activity) was also prepared and labeled with a luminescent metal chelate, as described above.

Example 2 - Binding Curve Experiment between Protein Tyrosine Kinase Product Tracer (PTK Tracer) and Anti-PTK Product (PY72) Antibody

A direct binding curve (showing luminescent metal chelate -labeled PY72 antibody binding to fluorescent acceptor labeled tracer) was generated by incubating serial dilutions of the labeled antibody (10 nM to 9.8 pM in two fold dilutions) with 1 nM fluorescent acceptor-labeled tracer (PTK labeled tracer; sequence F-ADE(pY)LIPQQS, where F is fluorescein and pY is a phosphorylated tyrosine, SEQ ID NO:1; note that the tracer is a phosphorylated tyrosine derivative of a protein tyrosine kinase (PTK) substrate) in FP dilution buffer (PanVera, Madison WI part #P2839). After a 30 minute incubation, the fluorescence polarization of each composition in the plate was read on a Tecan Ultra plate reader using a 485 nm excitation filter (20 nm bandpass) and 535 nm emission filters (25 nm bandpass). Data was collected using 10 flashes per well and a 40 μs integration time. The antibody was seen to bind to the tracer with an EC50 of slightly more than 1 nM. See FIG. 9.

A similar binding curve was performed with a luminescent metal chelated-labeled anti-pSer antibody and a fluorescent acceptor-labeled tracer (STK labeled tracer, sequence F-GRPRTS(pS)FAEG, where F is a fluorescein and pS is a phosphorylated serine, SEQ ID NO:2; note that the tracer is a phosphorylated serine derivative of a S/T kinase (STK) substrate).

Example 3 - Competition Curve between Labeled Kinase Product Tracer and Unlabeled Kinase Product

A competition curve to show that the disruption of the antibody-tracer interaction could be monitored by both fluorescence polarization and time-resolved RET from the same sample was performed by incubating serial dilutions (10 μM to 19.5 nM in two-fold dilutions) of an unlabeled phosphotyrosine-containing peptide competitor (ADE(pY)LIPQQS, where pY is a phosphorylated tyrosine, SEQ ID NO:3) in the presence of 10 nM Tb-chelate labeled PY72 antibody and 1 nM labeled PTK labeled tracer, as described above. After a 30 minute incubation, the plate was read on a Tecan Ultra plate reader. Fluorescence polarization was measured using a 485 nm excitation filter (20 nm bandpass) and 535 nm emission filters (25 nm bandpass). Time-resolved RET was measured using a 340 nm excitation filter (35 nm bandpass) and 495 nm (10 nm bandpass) and 520 nm (25 nm bandpass) filters using a 200 μs integration window after a 100 μs post-flash delay with 10 flashes per well. The time-resolved RET value (ratio) was calculated by dividing the 520 nm signal by the 495 nm signal. The shapes of the curves generated by TR-RET or FP were seen to nearly overlap, indicating that the presence of a phosphopeptide (such as that generated by a kinase reaction) could be detected and quantitated using FP or TR-RET, or both. See FIG. 10.

Example 4 - Screening of Test Compounds as Modulators of Kinase Activity using Multimode FP and TR-RET Measurements

A chemical library screen to identify inhibitors of Lyn B Kinase, a member of the SRC family of protein tyrosine kinase (PTK) enzymes, was performed. The kinase reaction was performed in the presence of 10 μM of a Prestwick library compound (test compound; Prestwick Library available from Prestwick Chemical, Inc., Washington DC) in 20 mM HEPES pH 7.5, 5 mM $MgCl_2$, 150 nM poly(Gly:Tyr, 4:1) protein tyrosine kinase substrate, and 10 μM ATP using 1 ng of Lyn B kinase per reaction. The kinase reaction was allowed to proceed for 1 hour at room temperature and then stopped by adding 100 mM EDTA to a final concentration of 5 mM in a total volume of 40 μl. To detect the presence of phosphopeptide product, 10 μl of a solution containing 20 nM Tb-chelate labeled PY72 antibody and 10 nM PTK labeled tracer was added to each well and incubated for an additional 30 min. The plate was then read on a Tecan Ultra plate reader in both fluorescence polarization and time-resolved RET measurement modes. Fluorescence polarization was measured using a 485 nm excitation filter (20 nm bandpass) and 535 nm emission filters (25 nm bandpass). Time-resolved RET was measured using a 340 nm excitation filter (35 nm bandpass) and 495 nm (10 nm bandpass) and 520 nm (25 nm bandpass) filters using a 200 μs integration window after a 100 μs post-flash delay with 10 flashes per well. The time-resolved RET value (ratio) was calculated by dividing the 520 nm signal by the 495 nm signal. Kinase inhibitors were identified by wells that showed high polarization or 520:495 TR-RET ratios. The results of the screen of approximately 750 compounds are shown in FIG. 11.

Example 5 - Conversion of FP Assay to Multiplex FP/TR-RET Assay

Because terbium-chelates are able to serve as donors to fluorophores such as fluorescein or rhodamine (and

derivatives thereof) in TR-RET assays, and because fluorescein and rhodamine have excellent properties for use in FP assays, it is a simple matter to modify an FP assay such that it can be read in a dual-mode FP/TR-RET manner by labeling, for example, a binding partner such as a receptor protein or an antibody with a fluorescent terbium chelate. The use of multiplex modes (e.g., both FP and TR-RET) allows verification of data and elimination of false positive or false negative results. In addition, assays that are problematic in either the FP mode or TR-RET mode may be converted to robust assays using the other mode.

An FP assay to detect phosphorylation of Ser133 on the cyclic-AMP response element binding protein (CREB) by CREB kinase (a serine kinase) was designed. The assay required the identification of a fluorescein-labeled kinase product tracer containing a phosphorylated serine. In addition, the assay required an anti-CREB pSer133 antibody (available from Cell Signaling Technologies, Beverly, MA) capable of binding the tracer. Four candidate tracer peptides were prepared, as shown below, and tested for binding to the anti-pSer133 antibody. The tracers differed in their length and in the position of the fluorophore on the peptide.

Tracer 1: Fluorescein-LRREILSRRP(pS)YRK (SEQ ID NO:4);
Tracer 2: Fluorescein-REILSRRP(pS)YRK (SEQ ID NO:5)
Tracer 3: Fluorescein-ILSRRP(pS)YRK (SEQ ID NO:6); and
Tracer 4: LRREILSRRP(pS)YRK-Fluorescein (SEQ ID NO:7).

When tested in direct binding to the antibody in FP mode, two tracers were seen to bind with sub-nM Kd affinities, but neither showed a change in polarization greater than 100 mP between the free and bound state. See FIG. 12A. The robustness of an FP assay is in part a function of the magnitude of this difference in polarization. As changes in polarization of greater than 30 mP, or greater than 50 mP, or greater than 100 mP, are generally preferred, an attempt was made to convert the assay to a TR-RET assay.

The anti-pSer133 antibody was labeled with CS124-DTPA-Phe-NCS*Tb (see Example 1 above) to yield an antibody with an average of 6.2 chelate molecules per antibody. When the four candidate tracer peptides were titrated separately against this labeled antibody, SEQ ID NO:7 was seen to bind with sub-nM affinity and a 32-fold change in TR-RET value between free and bound forms. See FIG. 12B.

## Example 6 - PKA Enzyme Titration Demonstrating Z'-Factor of TR-RET Assay

PKA (a serine kinase) was serially diluted across 24 wells of a 384 well plate and reacted with 1 μM peptide PKA substrate (LRRETLSRRPSYRK, SEQ ID NO:8) in 50 mM Tris (pH 7.5) containing 10 mM $MgCl_2$, 50 μM $NaVO_4$, and 5 μM ATP. The final reaction volume was 10 μL per well. The reactions were allowed to proceed for 90 minutes at room temperature, after which a 10 μL quench/detection solution (containing labeled tracer identified in Example 5 above), Tb-chelate-labeled anti-pSer133 antibody, and EDTA) was added. The plate was covered and incubated at room temperature for 2 hours. The plate was then read on a TECAN Ultra 384 fluorescence plate reader using a 340/35 nm excitation filter and 520/25 and 495/10 nm emission filters (Chroma Technology Corp.). Data was collected using 10 flashes per well with a 100 μs delay and 200 μs integration window. See FIG. 13A.

To assess assay robustness, a Z' value was determined from 48 20 μL wells containing Tb-chelate labeled anti-pSer133 antibody and labeled tracer (see above) in the presence (24 wells; "low signal" controls) or absence (24 wells, "high signal" controls) of 2.5 μM unlabeled tracer. The plate was covered and incubated for 2 hours at room temperature. The plate was then read on a TECAN Ultra 384 fluorescence plate reader using the parameters described above. The Z'-valuc was 0.92. See FIG. 13B.

## Example 7 - Conversion of Nuclear Receptor FP Assay to Multiplex FP/TR-RET Assay

To demonstrate the generality of the ability to convert FP assays to FP/TR-RET assays using terbium chelates, an Estrogen Receptor β (ER-β) FP competition assay was converted by directly labeling the ER receptor with an amine-reactive terbium chelate; see Example 1 above. In the FP assay, displacement of a fluorescein-labeled tracer by a competitor causes a change in the observed polarization from high to low. In the TR-RET assay, the amount of labeled tracer bound to receptor is measured by RET between the terbium chelate on the receptor and the fluorescein on the tracer. In the absence of a competitor the RET signal is high, and as the competitor displaces the tracer this signal decreases. 12.5 nM unlabeled (see FIG. 14A) or Tb-chelate labeled ER-β protein see FIG. 14B) were incubated with 1 nM labeled tracer (Fluormone ES2 (PanVera, Madison WI part#P2613)) and titrated with serial dilutions of unlabeled estradiol, a known ER-β ligand. Both FP and TR-RET assays showed similar EC50 values for the competition curve. In addition, the TR-RET assay offers the advantage that it could be re-formatted, with similar results expected, using limiting concentrations of receptor and excess concentrations of tracer.

Example 8 - Conversion of EGFR Kinase FP Assay to Multiplex FP/TR-RET Assay

The general method identified in Example 7 was used to screen for inhibitors of Epidermal Growth Factor Receptor (EGFR) Kinase (a protein tyrosine kinase) using the LOPAC (Sigma #LO1280) compound library. Hits identified in both readout modes were all seen to be true hits, whereas hits that showed discrepancy between readout modes were seen to be false. These results indicate that by multiplexing readout modes within an assay, one can significantly improve the integrity of the determined results.

Anti p-Tyr antibody (anti-pY20 available from Zymed) was concentrated to 5 mg/mL in 100 mM sodium carbonate buffer, pH 9.5. CS124-DTPA-Phe-NCS*Tb (Tb-chelate) was added at a 5 to 40-fold molar excess relative to antibody, and the reaction incubated at room temperature for 4 hours with light vortexing every 30 minutes. After 4 hours, the antibody was dialyzed twice against PBS to remove unreacted and/or hydrolyzed chelate. The amount of chelate bound to the antibody was quantitated by the absorbance of the CS124 moiety at 343 nm ($E_{340}$ = 11,440 $M^{-1}cm^{-1}$), and the amount of antibody quantitated by its absorbance at 280 nm ($E_{280}$ = 210,000 $M^{-1}cm^{-1}$), correcting for the absorbance of the CS124 at 280 nM (1.1 x its absorbance at 343 nM). See FIG. 15.

To determine whether labeling of the antibody affected its affinity for a fluorescein-labeled phosphopeptide tracer (see Example 2), binding curves were performed as previously described. At a labeling ratio of less than 9 chelates per antibody, the affinity for the tracer was seen to vary by less than 2-fold. See FIG. 16.

Epidermal Growth Factor Receptor (EGFR) Tyrosine Kinase (available from PanVera, Madison WI, #P2628) was screened for activity against the LOPAC$^{1280™}$ (Sigma #LO1280) library (containing 1280 compounds) in 10 μL reaction volume (20 μL detection volume) in Coming low-volume 384-well plates (part #3676). The kinase) reaction was performed in the presence of 10 μM library compound under the following reaction conditions: 20 mM BEPES pH 7.5, 5 mM $MgCl_2$, 2mM $MnCl_2$, 0.05mM $Na_3VO_4$, 1mM DTT, 150 nM poly(GlyTyr) 4:1 poly-GT tyrosine kinase substrate, and 10 μM ATP using 0.1. unit of kinase per reaction. The reaction was allowed to proceed for 90 minutes at 30°C, after which a 10 μl solution of a 20 mM EDTA, 8 nM Tb-labeled anti-ptyr (anti pY72 antibody; see Examples 1 and 2) and 4 nM PTK labeled-tracer (see Example 2 above) in TR-RET dilution buffer (PanVera, Madison WI part#PV3152) were added. The quenched reactions were then allowed to incubate for 1 hour at room temperature, after which they were read on a Tecan Ultra plate reader. Fluorescence Polarization was measured using a 485 nm excitation filter (20 nm bandpass) and 535 nm emission filters (25 nm bandpass). Time Resolved RET was measured using a 340 nm excitation filter (35 nm bandpass) and two emission filters; a 495nm with a 10 nm band pass for a reference peak and 520 nm with a 25mn band pass for signal change measurement, using a 200 μs integration window following a 100 μs post-flash delay. TR-RET filters were from Chroma Technology Corp. TR-RET values (ratios) were determined by dividing the intensity of the sample at 520 nm by the intensity of the sample at 495 nm. See FIG. 17.

Data from the FP and TR-RET reads were normalized and plotted on orthogonal axes. See FIG. 18. The difference in the percent inhibition as determined by FP and TR RET was determined. Four compounds that fell outside of three standard deviations from this average (CB1954, GW5074, Ergocristine, Pyrocatechol) were identified for further analysis. In addition, two compounds (Tyrophostin AG 1478, GW2974) showing strong correlation between detection modes and strong inhibition were also selected for follow-up profiling.

The two identified inhibitors (Tyrphostin AG1478 and GW2974, which are known inhibitors of EGFR kinase) were assayed in a series of 3-fold dilutions, and the four poorly-correlating compounds in a series of two-fold dilutions, against EGFR kinase under conditions as described in the library screen. Follow-up screening identified GW2974 as the more potent inhibitor, with an EC50 of about 10-fold less than that seen for AG1478. See FIG. 19.

To demonstrate the ability of the TR-RET detection mode to identify true hits even in the presence of interfering background fluorescence (a useful criteria when identifying either hits that are intrinsically fluorescent, or when screening libraries of pooled compounds in which the presence of a fluorescent compound could mask the presence of a hit), the assay was performed against a dilution series of the inhibitor Tyrphostin AG1478 in the presence of 10 nM fluorescein. The TR-RET data was seen to be impervious to the presence of the background fluorescence signal, whereas the FP data was severely compromised. See FIG. 20.

Two compounds that showed poor correlation between the FP and TR-RET detection modes, GW5074 and Ergocristine, were seen to precipitate, suggesting that the spurious signal in the FP detection mode was likely an artifact of light scatter. Because the signal due to scatter has a short lifetime, it does not affect the TR-RET reading mode. See FIG. 21.

Two other compounds that showed poor correlation, CB-1954 and Pyrocatechol, were re-assayed and neither was seen to be an inhibitor. An examination of the screen showed that these compounds were in adjacent wells of the assay plate, suggesting a systematic error that led to the spurious results. See FIG. 22.

To assess the concentration of phosphorylated kinase product required to give a detectable change in signal, a serial dilution of an unlabeled phosphorylated PTK tracer (as competitor product to tracer) was incubated with 4 nM Tb-chelate labeled anti-pTyr antibody and 2 nM fluorescein-labeled PTK tracer in TR-RET dilution buffer (see above). The plate was then read in both FP and TR-RET modes as described previously. The amount of competitor required

for half-maximal signal change was seen to be nearly identical between assay modes, indicating that both assays had similar sensitivities. See FIG 23.

To assess assay robustness, 60 wells containing 4 nM Tb-chelate labeled anti-pTyr antibody and 2 nM fluorescein-labeled tracer (the "high value" controls), and 60 wells containing the same components in addition to 1 uM competitor peptide (the "low value" controls) were read in both FP and TR-RET detection modes as described previously. Z' values were calculated according to Zhang et al., "A Simple Statistical Parameter for Use in Evaluation and Validation of High Throughput Screening Assays," Journal of Biomolecular Screening 4(2):67-73 (1999). The Z'-factor was seen to be > 0.8 for each assay mode. See FIG. 24.

Example 9 - Multiplex FP/TR-RET Assa using an Eu(III)-Chelate-labeled Binding Partner

Binding partners labeled with Eu-chelates can also be used in the methods of the present invention.

Buropium(III)-chelate labeled PY72 (anti-phosphotyrosine) antibody (see Example 1) was prepared as follows. To 50 μL of a 28.4 μM solution of PY72 antibody in phosphate-buffered saline (PBS) was added 1 μL of 21.25 mM SPDP (N-Succinimidyl 3-(2-pyridyldithio) propionate, Pierce Chemical Company) in DMSO. After a one hour reaction at room-temperature, 50 μL of 50 mM dithiothreitol (DTT) in 100 mM sodium acetate buffer, pH 4,5, was added and the reaction allowed to incubate an additional 30 minutes at room temperature. The reaction was then dialyzed twice for two hours each against 1 L degassed PBS buffer. After dialysis, 8 μL of a solution containing 4.2 mM TTHA-AMCA-(2-amioethyl)maleimde and 10 mM $EuCl_3$ in 1 M Tris, pH 8.0, was added to the antibody solution and allowed to incubate for 2 hours at room temperature. The labeled antibody was then dialyzed twice (first for two hours, then overnight) to remove excess and unreacted chelate.

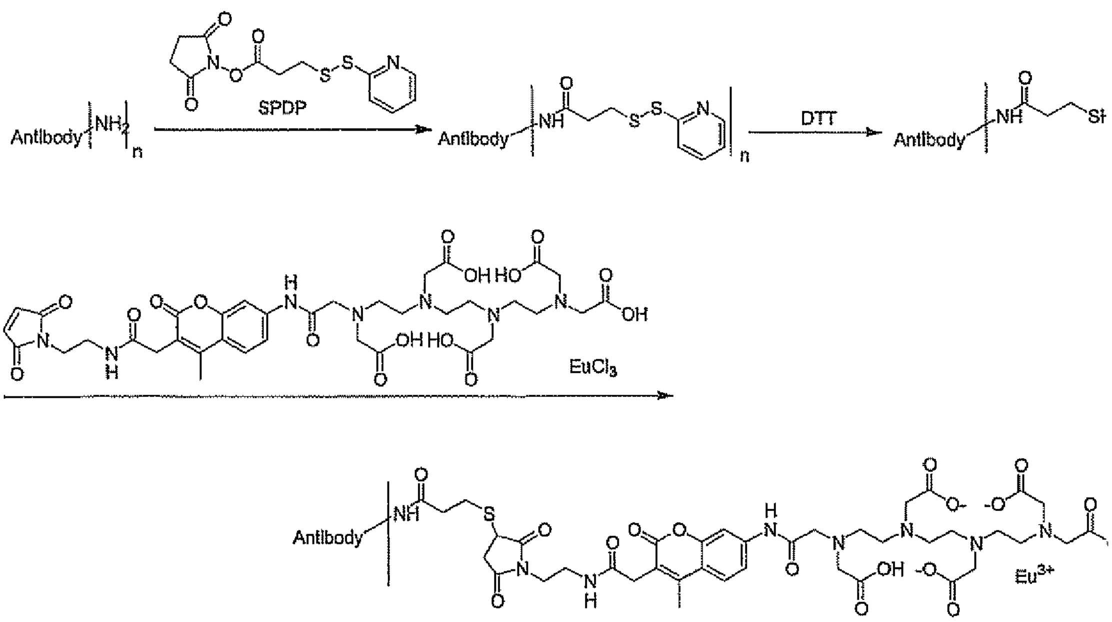


Labeling of PY72 antibody using SPDP and Eu-TTHA-AMCA-(2-aminoethyl)maleimide.

A competition curve to show that the disruption of an Eu-chelate labeled antibody-labeled tracer interaction by an unlabeled phosphopeptide (e.g., a product of a protein kinase enzymatic reaction) could be measured by fluorescence polarization and/or time-resolved RET from the same sample was performed by incubating serial dilutions of an unlabeled phosphotyrosine-containing peptide competitor (2 μM to 1 nM in two-fold dilutions; in the presence of 5 nM Eu-chelate labeled PY72 antibody and 1 nM luminescent tracer in FP dilution buffer (PanVera, Madison WI, Part #P2839). The luminescent labeled tracer was Alexa Fluor 633-CADE(pY)LBPQQS (SEQ ID NO:10), a peptide in which the C5 maleimide derivative of Alexa Fluor 633 (Molecular Probes, Eugene OR, Part #A20342) had been coupled to the terminal cysteine of the peptide using standard procedures (following the protocol included with the Alexa Fluor dye) and purified via HPLC using standard procedures. The peptide (CADE(pY)LIPQQS; SEQ ID NO:9) had been ordered by AnaSpee, San Jose CA. Alexa Fluor 633 has a maximum excitation wavelength of approximately 622 nm and a maximum emission wavelength of approximately 640 nm in aqueous solution. After a 30 minute incubation, the plate was read on a Tecan Ultra plate

reader in both FP and TR-RET formats. Fluorescence polarization was measured using a 590 nm excitation filter (20 nm bandpass) and 650 nm emission filters (40 nm bandpass). Time-resolved RET was measured using a 340 nm excitation filter (35 nm bandpass) and 615 nm (10 nm bandpass) and 665 nm (10 nm bandpass) emission filters using a 200 $\mu$s integration window after a 100 $\mu$s post-flash delay with 10 flashes per well. The time-resolved RET value (ratio) was calculated by dividing the 665 nm signal by the 615 nm signal. The shape of the curves generated by TR-RET or FP were seen to nearly overlap, indicating that the presence of a competitor phosphopeptide (such as that generated by a kinase reaction) could be detected and quantitated using either FP or TR-RET modes. See FIG. 25.

Example 10 - Detection of Histidine-tagged Proteins using Multiplex Modes

A multiplex system for the detection of His-tagged proteins or peptides was developed. The basis of the assay was a competition between a Histidine-tagged analyte protein and a tracer consisting of fluorescein linked to a hexahistidine peptide for a terbium-chelate labeled anti-His-tag antibody. In the absence of analyte protein or peptide, the fluorescein-labeled hexahistidine peptide associates with the anti-His-tag antibody, and this interaction can be detected by TR-RET or FP. In the presence of increasing amounts of analyte protein, this tracer-antibody interaction is disrupted and the TR-RET signal or fluorescence polarization of the tracer decreases. Fluorescein-His6 peptide (fluarescein-HHHHHH, the "luminescent tracer;" SEQ ID NO:11) was synthesized by a commercial supplier (ResGen, Huntsville AL) and used as supplied. A commercial monoclonal antibody specific for the hexahistidine tag (Part MCA1396, Serotec, Raleigh, NC) was purchased and used as supplied with no additional purification. 0.25 mg antibody was concentrated in 100 mM sodium carbonate buffer, pH 9.5, to a final volume of 50 uL (5 mg / mL final concentration of antibody). To label the antibody, 30 ug of CS124-DTPA-Phe-NCS-Tb (a 20-fold molar excess relative to antibody) was added and the reaction allowed to proceed at room temperature for 4 hours with light vortexing every 30 minutes. After 4 hours, the antibody was dialyzed twice versus PBS to remove unreacted and/or hydrolyzed chelate. The amount of chelate bound to the antibody was quantitated by the absorbance of the CS124 moiety at 343 nm ($E_{340}$ = 11,440 $M^{-1}cm^{-1}$), and the amount of antibody quantitated by its absorbance at 280 nm ($B_{280}$ = 210,000 $M^{-1}cm^{-1}$), correcting for the absorbance of the CS124 at 280 nM (1.1 x its absorbance at 343 nM). From these measurements an average of 7.7 chelates per antibody was determined. The labeled antibody was seen to be stable for at least 6 months with no noticeable loss in performance.

A competitive binding assay was performed with 20 nM antibody and 2 nM tracer, with titration of increasing amounts of His-tagged peptide (sequence: Biotin-KGGHHHHHH, source: ResGen; SEQ ID NO:12) ranging from 3 uM to 1.5 nM in two-fold dilutions. The assay components were mixed in FP Dilution buffer (see above) and read after a 30 minute incubation on a Tecan Ultra plate reader using a 340 nm excitation filter (35 nm bandpass) and a 520 nm emission filter (25 nm bandpass). Data were collected using a 200 $\mu$s integration window after a 100 $\mu$s post-flash delay, with 10 flashes per well. The data are shown in FIG. 26A and B.

Further embodiments are described in the following numbered paragraphs.

1. A method for measuring the effect of a test compound on binding between a first binding partner and a second binding partner, said method comprising:

a) contacting a first binding partner, a second binding partner, and a test compound to form a test sample, wherein said first binding partner comprises a luminescent metal complex, wherein said first binding partner and said second binding partner are capable of binding to one another to form a complex, and wherein said second binding partner comprises a fluorescent acceptor moiety;
b) exposing said test sample to polarized light and measuring the polarization of fluorescent emission from said test sample; and
c) exposing said test sample to light having a wavelength in the range from 250 nm to 750 nm and measuring the fluorescence emission from said test sample;

wherein said test compound is identified as affecting binding between said first binding partner and said second binding partner when the fluorescence polarization measurement or the fluorescence emission measurement of said test sample is different from the fluorescence polarization measurement or the fluorescence emission measurement of a corresponding control sample lacking said test compound.

2. A method for measuring the effect of a test compound on binding between a first binding partner and a second binding partner, said method comprising:

a) contacting a first binding partner, a second binding partner, and a test compound to form a test sample, wherein said first binding partner comprises a fluorescent acceptor moiety, wherein said first binding partner

and said second binding partner are capable of binding to one another to form a complex, and wherein said second binding partner comprises a luminescent metal complex;
b) exposing said test sample to polarized light and measuring the polarization of fluorescent emission from said test sample; and
c) exposing said test sample to light having a wavelength in the range from 250 nm to 750 nm and measuring the fluorescence emission from said test sample;

wherein said test compound is identified as affecting binding between said first binding partner and said second binding partner when the fluorescence polarization measurement or the fluorescence emission measurement of said test sample is different from the fluorescence polarization measurement or the fluorescence emission measurement of a corresponding control sample lacking said test compound.

3. The method according to paragraph 1 or 2, wherein said first binding partner and said second binding partner are independently selected from the group consisting of a polypeptide; a polynucleotide, a lipid, a polysaccharide, a hormone, and a small organic compound.

4. The method according to paragraph 3, wherein said polypeptide is an antibody or antibody fragment.

5. The method according to paragraph 1 or 2, wherein said step b) and said step c) are performed simultaneously.

6. The method according to paragraph 1 or 2, wherein said step b) is performed prior to said step c).

7. The method according to paragraph 1 or 2, wherein said step b) is performed after said step c).

8. The method of paragraph 1 or 2, wherein said fluorescent acceptor moiety is selected from the group consisting of fluorescein, rhodamine, GFP, GFP derivatives, FITC, 5-FAM, 6-FAM, 7-hydroxycoumarin-3-carboxamide, 6-chloro-7-hydroxycoumarin-3-carboxamide, fluorescein-5-isothiocyanate, dichlorotriazinylaminofluorescein, tetramethylrhodamine-5-isothiocyanate, tetramethylrhodamine-6-isothiocyanate, succinimidyl ester of 5-carboxyfluorescein, succinimidyl ester of 6-carboxyfluorescein, 5-carboxytetramethylrhodamne, 6-carboxymethylrhodamine, and 7-amino-4-methylcoumarin-3-acetic acid.

9. The method of paragraph 1 or 2, wherein said luminescent metal complex is a lanthanide metal complex.

10. The method of paragraph 9, wherein said lanthanide metal complex comprises an organic antenna moiety, a metal liganding moiety and a lanthanide metal ion.

11. The method of paragraph 10, wherein said lanthanide metal ion is selected from the group consisting of: Sm (III), Ru(III), Eu(III), Gd(III), Tb(III), and Dy(III).

12. The method of paragraph 10, wherein said organic antenna moiety is selected from the group consisting of: rhodamine 560, fluorescein 575, fluorescein 590, 2-quinolone, 4-quinolone, 4-trifluoromethylcoumarin (TFC), 7-diethyl-amino-coumearin -3-carbohydrazide, 7-amina-4-methyl-2-coumarin (carbostyril 124), 7-animo-4-methyl-2-coumarin (coumarin 120), 7-amino-4-trifluoromethyl-2-coumarin (coumarin 124), and aminomethyltrimethylpsoralen.

13. The method of paragraph 10, wherein said metal liganding moiety is a metal cheating moiety selected from the group consisting of: EDTA, DTPA, TTHA, DOTA, NTA, HDTA, DTPP, EDTP, HDTP, NTP, DOTP, DO3A, DOTAGA, and ROTA.

14. The method of paragraph 10, wherein said lanthanide metal complex has a structure:

$$-L_n-A-S_n-C_M,$$

or

$$-L_n-C_M-S_n-A,$$

wherein A represents an organic antenna moiety;

L represents a linker;
S represents a spacer;
n can be 0 or 1;
C represents a metal chelating moiety; and
M represents a lanthanide metal ion coordinated to C.

15. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in the presence of a potential modulator of said enzymatic activity;
b) contacting said enzyme, said substrate, and said potential modulator with a first binding partner and a luminescent tracer to form a test sample, wherein said first binding partner has binding specificity for said product as compared to said substrate, wherein said first binding partner comprises a luminescent metal complex, wherein said first binding partner is capable of binding said luminescent tracer, and wherein said luminescent tracer comprises a fluorescent acceptor moiety;
c) exposing said test sample to polarized light and measuring the polarization of fluorescent emission from said test sample; and
d) exposing said test sample to light having a wavelength in the range from 250 nm to 750 nm and measuring the fluorescence emission from said test sample;

wherein said potential modulator is identified as a modulator of said enzymatic activity of said enzyme when said fluorescence polarization measurement or said fluorescence emission measurement of said test sample is different from the fluorescence polarization measurement or fluorescence emission measurement, respectively, of a corresponding control sample lacking said potential modulator.

16. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in the presence of a potential modulator of said enzymatic activity;
b) contacting said enzyme, said substrate, and said potential modulator with a first binding partner and a luminescent tracer to form a test sample, wherein said first binding partner has binding specificity for said product as compared to said substrate, wherein said first binding partner is capable of binding said luminescent tracer, wherein said first binding partner comprises a fluorescent acceptor moiety, and wherein said luminescent tracer comprises a luminescent metal complex;
c) exposing said test sample to polarized light and measuring the polarization of fluorescent emission from said test sample; and
d) exposing said test sample to light having a wavelength in the range from 250 nm to 750 nm and measuring the fluorescence emission from said test sample;

wherein said potential modulator is identified as a modulator of said enzymatic activity of said enzyme when said fluorescence polarization measurement or said fluorescence emission measurement of said test sample is different from the fluorescence polarization measurement or fluorescence emission measurement, respectively, of a corresponding control sample lacking said potential modulator.

17. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in the presence of a potential modulator of said enzymatic activity;
b) contacting said enzyme, said substrate, and said potential modulator with a first binding partner and a luminescent tracer to form a test sample, wherein said first binding partner has binding specificity for said substrate as compared to said product, wherein said first binding partner is capable of binding said luminescent tracer, wherein said first binding partner comprises a luminescent metal complex, and wherein said luminescent tracer comprises a fluorescent acceptor moiety;
c) exposing said test sample to polarized light and measuring the polarization of fluorescent emission from said test sample; and

d) exposing said test sample to light having a wavelength in the range from 250 nm to 750 nm and measuring the fluorescence emission from said test sample;

wherein said potential modulator is identified as a modulator of said enzymatic activity of said enzyme when said fluorescence polarization measurement or said fluorescence emission measurement of said test sample is different from the fluorescence polarization measurement or fluorescence emission measurement, respectively, of a corresponding control sample lacking said potential modulator.

18. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in the presence of a potential modulator of said enzymatic activity;
b) contacting said enzyme, said substrate, and said potential modulator with a first binding partner and a luminescent tracer to form a test sample, wherein said first binding partner has binding specificity for said substrate as compared to said product, wherein said first binding partner is capable of binding said luminescent tracer, wherein said first binding partner comprises a fluorescent acceptor moiety, and wherein said luminescent tracer comprises a luminescent metal complex;
c) exposing said test sample to polarized light and measuring the polarization of fluorescent emission from said test sample; and
d) exposing said test sample to light having a wavelength in the range from 250 nm to 750 nm and measuring the fluorescence emission from said test sample;

wherein said potential modulator is identified as a modulator of said enzymatic activity of said enzyme when said fluorescence polarization measurement or said fluorescence emission measurement of said test sample is different from the fluorescence polarization measurement or fluorescence emission measurement, respectively, of a corresponding control sample lacking said potential modulator.

19. The method of paragraph 15, 16, 17, or 18, wherein said enzymatic activity is selected from the group consisting of kinase activity, phosphatase activity, glucuronidase activity, prenylation, glycosylation, methylation, demethylation, acylation, acetylation, ubiquitination, sulfation, proteolysis, nuclease activity, nucleic acid polymerase activity, nucleic acid reverse transcriptase activity, nucleotidyl transferase activity and polynucleotide translation activity.

20. The method of paragraph 1, 2, 15, 16, 17, or 18, wherein the lifetime of said test sample's fluorescence emission is from about 500 picoseconds to about 100 nanoseconds.

21. The method of paragraph 1, 2, 15, 16, 17, or 18, wherein said difference in said fluorescence polarization measurement of said test sample as compared to said control sample is from about 30 mP to about 450 mP.

22. The method of paragraph 1, 2, 15, 16, 17, or 18, wherein said difference in said fluorescence polarization measurement of said test sample as compared to said control sample is from about a 10% to about 10,000% increase or decrease.

23. The method of paragraph 1, 2, 15, 16, 17, or 18, wherein the fluorescence emission of said test sample is measured at two wavelengths.

24. The method of paragraph 23, wherein a ratio of said fluorescence emission of said test sample at said two wavelengths is calculated.

25. An article of manufacture comprising:

a) packaging material;
b) a first binding partner comprising a luminescent metal complex; and
c) a second binding partner, wherein said second binding partner specifically binds said first binding partner and wherein said second binding partner comprises a fluorescent acceptor moiety.

26. An apparatus comprising:

a) a sample chamber;
b) means for generating plane polarized light to illuminate said sample chamber;
c) means for detecting polarized light emitted from said sample chamber;
d) means for illuminating said sample chamber with light having a wavelength from 250 nm to 750 nm; and
e) means for detecting light emitted from said sample chamber.

27. The apparatus of paragraph 26, wherein said means for generating plane polarized light to illuminate said sample chamber and said means for illuminating said sample chamber with light having a wavelength from 250 nm to 750 nm are arranged so that both of said illumination means simultaneously illuminate said sample chamber with said plane polarized light and said light having a wavelength from 250 nm to 750 nm, respectively.

28. The apparatus of paragraph 26, wherein said means for detecting polarized light emitted from said sample chamber and said means for detecting light emitted from said sample chamber are arranged so that both of said detection means simultaneously detect said polarized light and said light emitted from said sample chamber.

29. A method for measuring the effect of a test compound on binding between a first binding partner and a second binding partner, said method comprising:

a) contacting a first binding partner, a second binding partner, and a test compound to form a test sample, wherein said first binding partner comprises a luminescent metal complex, wherein said first binding partner and said second binding partner are capable of binding to one another to form a complex, and wherein said second binding partner comprises a fluorescent acceptor moiety; and
b) exposing said test sample to polarized light and measuring the polarization of fluorescent emission from said test sample;

wherein said test compound is identified as affecting binding between said first binding partner and said second binding partner when the fluorescence polarization measurement of said test sample is different from the fluorescence polarization measurement of a corresponding control sample lacking said test compound.

30. A method for measuring the effect of a test compound on binding between a first binding partner and a second binding partner, said method comprising:

a) contacting a first binding partner, a second binding partner, and a test compound to form a test sample, wherein said first binding partner comprises a fluorescent acceptor moiety, wherein said first binding partner and said second binding partner are capable of binding to one another to form a complex, and wherein said second binding partner comprises a luminescent metal complex; and
b) exposing said test sample to polarized light and measuring the polarization of fluorescent emission from said test sample;

wherein said test compound is identified as affecting binding between said first binding partner and said second binding partner when the fluorescence polarization measurement of said test sample is different from the fluorescence polarization measurement of a corresponding control sample lacking said test compound.

31. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in the presence of a potential modulator of said enzymatic activity;
b) contacting said enzyme, said substrate, and said potential modulator with a first binding partner and a luminescent tracer to form a test sample, wherein said first binding partner has binding specificity for said product as compared to said substrate, wherein said first binding partner comprises a luminescent metal complex, wherein said first binding partner is capable of binding said luminescent tracer, and wherein said luminescent tracer comprises a fluorescent acceptor moiety; and
c) exposing said test sample to polarized light and measuring the polarization of fluorescent emission from said test sample;

wherein said potential modulator is identified as a modulator of said enzymatic activity of said enzyme when said fluorescence polarization measurement of said test sample is different from the fluorescence polarization measurement of a corresponding control sample lacking said potential modulator.

32. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in the presence of a potential modulator of said enzymatic activity;
b) contacting said enzyme, said substrate, and said potential modulator with a first binding partner and a luminescent tracer to form a test sample, wherein said first binding partner has binding specificity for said product as compared to said substrate, wherein said first binding partner is capable of binding said luminescent tracer, wherein said first binding partner comprises a fluorescent acceptor moiety, and wherein said luminescent tracer comprises a luminescent metal complex; and
c) exposing said test sample to polarized light and measuring the polarization of fluorescent emission from said test sample;

wherein said potential modulator is identified as a modulator of said enzymatic activity of said enzyme when said fluorescence polarization measurement of said test sample is different from the fluorescence polarization measurement of a corresponding control sample lacking said potential modulator.

33. A method for identifying a modulator of an enzymatic activity, said method comprising:

a) contacting an enzyme with a substrate for said enzyme, said contacting carried out under conditions effective for an enzymatic activity of said enzyme to for a product from said substrate, said contacting carried out in the presence of a potential modulator of said enzymatic activity;
b) contacting said enzyme, said substrate, and said potential modulator with a first binding partner and a luminescent tracer to form a test sample, wherein said first binding partner has binding specificity for said substrate as compared to said product, wherein said first binding partner is capable of binding said luminescent tracer, wherein said first binding partner comprises a luminescent metal complex, and wherein said luminescent tracer comprises a fluorescent acceptor moiety; and
c) exposing said test sample to polarized light and measuring the polarization of fluorescent emission from said test sample;

wherein said potential modulator is identified as a modulator of said enzymatic activity of said enzyme when said fluorescence polarization measurement of said test sample is different from the fluorescence polarization measurement of a corresponding control sample lacking said potential modulator.

34. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in the presence of a potential modulator of said enzymatic activity;
b) contacting said enzyme, said substrate, and said potential modulator with a first binding partner and a luminescent tracer to form a test sample, wherein said first binding partner has binding specificity for said substrate as compared to said product, wherein said first binding partner is capable of binding said luminescent tracer, wherein said first binding partner comprises a fluorescent acceptor moiety, and wherein said luminescent tracer comprises a luminescent metal complex; and
c) exposing said test sample to polarized light and measuring the polarization of fluorescent emission from said test sample;

wherein said potential modulator is identified as a modulator of said enzymatic activity of said enzyme when said fluorescence polarization of said test sample is different from the fluorescence polarization measurement of a corresponding control sample lacking said potential modulator.

35. A composition comprising a first binding partner and a second binding partner, wherein said first binding partner comprises a luminescent metal complex and said second binding partner comprises a fluorescent acceptor moiety.

36. A composition comprising a first binding partner and a second binding partner, wherein said first binding partner comprises a fluorescent acceptor moiety and said second binding partner comprises a luminescent metal complex.

37. A method for measuring the effect of a test compound on binding between a first binding partner and a second binding partner, said method comprising:

a) contacting a first binding partner, a second binding partner, and a test compound to form a test sample, wherein said first binding partner comprises a luminescent metal complex, wherein said first binding partner and said second binding partner are capable of binding to one another to form a complex, and wherein said test compound comprises a fluorescent acceptor moiety;
b) exposing said test sample to polarized light and measuring the polarization of fluorescent emission from said test sample; and
c) exposing said test sample to light having a wavelength in the range from 250 nm to 750 nm and measuring the fluorescence emission from said test sample;

wherein said test compound is identified as affecting binding between said first binding partner and said second binding partner when the fluorescence polarization measurement or the fluorescence emission measurement of said test sample is different from the fluorescence polarization measurement or the fluorescence emission measurement of a corresponding control sample lacking said test compound.

38. A method for measuring the effect of a test compound on binding between a first binding partner and a second binding partner, said method comprising:

a) contacting a first binding partner, a second binding partner, and a test compound to form a test sample, wherein said first binding partner comprises a fluorescent acceptor moiety, wherein said first binding partner and said second binding partner are capable of binding to one another to form a complex, and wherein said test compound comprises a luminescent metal complex;
b) exposing said test sample to polarized light and measuring the polarization of fluorescent emission from said test sample; and
c) exposing said test sample to light having a wavelength in the range from 250 nm to 750 nm and measuring the fluorescence emission from said test sample;

wherein said test compound is identified as affecting binding between said first binding partner and said second binding partner when the fluorescence polarization measurement or the fluorescence emission measurement of said test sample is different from the fluorescence polarization measurement or the fluorescence emission measurement of a corresponding control sample lacking said test compound.

39. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in the presence of a potential modulator of said enzymatic activity, and wherein said substrate comprises a fluorescent acceptor moiety;
b) contacting said enzyme, said substrate, and said potential modulator with a first binding partner and a tracer to form a test sample, wherein said first binding partner has binding specificity for said product as compared to said substrate, wherein said first binding partner comprises a luminescent metal complex, and wherein said first binding partner is capable of binding said tracer;
c) exposing said test sample to polarized light and measuring the polarization of fluorescent emission from said test sample; and
d) exposing said test sample to light having a wavelength in the range from 250 nm to 750 nm and measuring the fluorescence emission from said test sample;

wherein said potential modulator is identified as a modulator of said enzymatic activity of said enzyme when said fluorescence polarization measurement or said fluorescence emission measurement of said test sample is different from the fluorescence polarization measurement or fluorescence emission measurement, respectively, of a corresponding control sample lacking said potential modulator.

40. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in

the presence of a potential modulator of said enzymatic activity, and wherein said substrate comprises a luminescent metal complex;
b) contacting said enzyme, said substrate, and said potential modulator with a first binding partner and a tracer to form a test sample, wherein said first binding partner has binding specificity for said product as compared to said substrate, wherein said first binding partner is capable of binding said tracer, and wherein said first binding partner comprises a fluorescent acceptor moiety;
c) exposing said test sample to polarized light and measuring the polarization of fluorescent emission from said test sample; and
d) exposing said test sample to light having a wavelength in the range from 250 nm to 750 nm and measuring the fluorescence emission from said test sample;

wherein said potential modulator is identified as a modulator of said enzymatic activity of said enzyme when said fluorescence polarization measurement or said fluorescence emission measurement of said test sample is different from the fluorescence polarization measurement or fluorescence emission measurement, respectively, of a corresponding control sample lacking said potential modulator.

41. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in the presence of a potential modulator of said enzymatic activity, and wherein said substrate comprises a fluorescent acceptor moiety;
b) contacting said enzyme, said substrate, and said potential modulator with a first binding partner and a tracer to form a test sample, wherein said first binding partner has binding specificity for said substrate as compared to said product, wherein said first binding partner is capable of binding said tracer, and wherein said first binding partner comprises a luminescent metal complex;
c) exposing said test sample to polarized light and measuring the polarization of fluorescent emission from said test sample; and
d) exposing said test sample to light having a wavelength in the range from 250 nm to 750 nm and measuring the fluorescence emission from said test sample;

wherein said potential modulator is identified as a modulator of said enzymatic activity of said enzyme when said fluorescence polarization measurement or said fluorescence emission measurement of said test sample is different from the fluorescence polarization measurement or fluorescence emission measurement, respectively, of a corresponding control sample lacking said potential modulator.

42. A method for identifying a modulator of an enzymatic activity, said method comprising:

a) contacting an enzyme with a substrate for said enzyme, said contacting carried out under conditions effective for an enzymatic activity of said enzyme to form a product from said substrate, said contacting carried out in the presence of a potential modulator of said enzymatic activity, and wherein said substrate comprises a luminescent metal complex;
b) contacting said enzyme, said substrate, and said potential modulator with a first binding partner and a tracer to form a test sample, wherein said first binding partner has binding specificity for said substrate as compared to said product, wherein said first binding partner is capable of binding said tracer, and wherein said first binding partner comprises a fluorescent acceptor moiety;
c) exposing said test sample to polarized light and measuring the polarization of fluorescent emission from said test sample; and
d) exposing said test sample to light having a wavelength in the range from 250 nm to 750 nm and measuring the fluorescence emission from said test sample;

wherein said potential modulator is identified as a modulator of said enzymatic activity of said enzyme when said fluorescence polarization measurement or said fluorescence emission measurement of said test sample is different from the fluorescence polarization measurement or fluorescence emission measurement, respectively, of a corresponding control sample lacking said potential modulator.

## Claims

**1.** A method for identifying a modulator of an enzymatic activity, the method comprising:

exposing to a light having a wavelength in the range from 250 nm to 750 nm a test sample comprising:

i) an enzyme, a substrate for the enzyme, a potential modulator, a first binding partner, and a luminescent tracer with the first binding partner is capable of binding, the first binding partner having specificity for either the substrate or a product of an enzymatic activity on the substrate, wherein one of the binding partner and the tracer includes a luminescent metal complex whilst the other includes a fluorescent protein, wherein the luminescent metal complex and fluorescent protein are capable of acting as resonance energy transfer pair, or
ii) an enzyme, a substrate, a potential modulator, and a first binding partner, the first binding partner having specificity for either the substrate or a product of an enzymatic activity on the substrate, wherein one of the binding partner and the substrate includes a luminescent metal complex whilst the other includes a fluorescent protein, wherein the luminescent metal complex and fluorescent protein are capable of acting as resonance energy transfer pair; and

measuring the fluorescence emission from the test sample;
wherein the potential modulator is identified as a modulator of the enzymatic activity of the enzyme when the fluorescence emission measurement of the test sample is different from the fluorescence emission measurement of a corresponding control sample lacking the potential modulator.

**2.** The method of claim 1 wherein the test sample is obtainable by:

contacting the enzyme with the substrate for the enzyme, where the contacting is carried out under conditions effective for an enzymatic activity of the enzyme to form a product from the substrate and where the contacting is carried out in the presence of a potential modulator of the enzymatic activity;
then contacting the enzyme, substrate, and potential modulator with the first binding partner and the tracer to form the test sample.

**3.** The method of claim 1 or claim 2 wherein a second substrate for the enzyme is contacted with the enzyme,optionally wherein the contacting further involvesa second binding partner having specificity for either the second substrate or a product of an enzymatic activity on the second substrate, wherein one of the second binding partner and the second substrate includes a luminescent metal complex whilst the other includes a fluorescent protein, wherein the luminescent metal complex and fluorescent protein are capable of acting as resonance energy transfer pair.

**4.** The method of claim 1 in which a plurality of substrates are contacted, independently, with a particular enzyme and potential modulator, to form a plurality of enzyme mixtures, each enzyme mixture then being contacted with an appropriate first binding partner and luminescent tracer to form a test sample.

**5.** The method of any of claims 1 to 4, wherein the first binding partner and, where present, the second binding partner are independently selected from the group consisting of a polypeptide, a polynucleotide, a lipid, a phospholipid, a polysaccharide, or an organic molecule,optionally wherein the polypeptide is an antibodyor antibody fragment e.g. the antibody is a polyclonal antibody, a monoclonal antibody, a humanised or chimeric antibody, a single chain Fv antibody fragment, a Fab fragment or a $F(ab)_2$ fragment, optionally wherein the tracer includes the purported epitope

recognized by the binding partner which is an antibody.

**6.** The method of any preceding claim wherein the test sample comprises an unlabeled tracer.

**7.** The method of any of claims 1 to 5 wherein the tracer includes a luminescent metal complex which optionally includes a metal liganding moiety and one or more lanthanide metal ions, optionally wherein the lanthanide metal ion is Sm (III), Ru(III), Eu(III), Gd(III), Tb(III) or Dy(III) and optionally wherein the a Tb(III) lanthanide metal ion or an Eu(III) lanthanide metal ion, e.g. wherein the fluorescent protein is a GFP or GFP mutant and the lanthanide metal complex is a Tb(III)-containing metal complex.

**8.** The method of any preceding claim wherein the enzymatic activity is selected from the group consisting of kinase activity, phosphatase activity, glucuronidase activity, prenylation, glycosylation, methylation, demethylation, acylation, acetylation, ubiquitination, sulfation, proteolysis, nuclease activity, nucleic acid polymerase activity, nucleic acid reverse transcriptase activity, nucleotidyl transferase activity, and polynucleotide translation activity.

**9.** The method of any preceding claim wherein the measuring of the fluorescence emission comprises measuring time resolved resonance energy transfer.

**10.** The method of any preceding claim wherein the fluorescent protein is a GFP or GFP mutant, optionally wherein wherein the mutant GFP is a GFP of Table 7 of US-A-6410255, namely a mutation listed in the following Table:

| ***Mutations*** | ***Common Name*** |
|---|---|
| S65 type | |
| S65T, S72A, N149K, M153T, I167T | Emerald |
| F64L, S65T, V163A | |
| F64L, S65T (EGFP) | EGFP |
| S65T | |
| Y66H type | |
| F64L, Y66H, Y145F, V163A | P4-3E |
| F64L, Y66H, Y145F | |
| Y66H, Y145F | P4-3 |
| Y66H | BFP |
| Y66W type | |
| S65A, Y66W, S72A, N146I, M153T, V163A | W1C |
| F64L, S65T, Y66W, N146I, M153T, V163A | W1B |
| Y66W, N146I, M153T, V163A | hW7 |
| Y66W | |
| T203Y type | |
| S65G, S72A, K79R, T203Y | Topaz |
| S65G, V68L, S72A, T203Y | 10C |
| S65G, V68L, Q69K, S72A, T203Y | h10C+ |
| S65G, S72A, T203H | |
| S65G, S72A, T203F | |

(continued)

| T203I type | |
|---|---|
| T203I, S72A, Y145F | Sapphire |
| T203I, T202F | H9 |

**11.** The method of any preceding claim wherein the luminescent metal complex includes a metal liganding moiety and one or more lanthanide metal ions, optionally wherein the lanthanide metal ion is Sm(III), Ru(III), Eu(III), Gd(III), Tb (III) or Dy(III) and optionally wherein the a Tb(III) lanthanide metal ion or an Eu(III) lanthanide metal ion, e.g. wherein the fluorescent protein is a GFP or GFP mutant and the lanthanide metal complex is a Tb(III)-containing metal complex.

**12.** The method of claim 7 or claim 11 wherein the lanthanide metal complex comprises an organic antenna moiety, a metal liganding moiety and a lanthanide metal ion, optionally wherein containing complex has one of the following structures:

$$-L_n-A-S_n-C_M,$$

or

$$-L_n-C_M-Sn-A,$$

wherein

A represents an organic antenna moiety, e.g. a polynuclear heterocyclic aromatic compound;
n is 0 or 1;
L represents a linker, e.g. L is -NH-CO-NH-; $-CO-(CH_2)_n-NH-$ where n=1 to 10; -NH-Ph-; $-NH-(CH_2)_n-$, wherein n=1 to 10; -CO-NH-; $-(CH_2)_n-NH-$ where n=1 to 10; $-CO-(CH_2)_n-NH-$, where n=1 to 10; or -CS-NH-;
S represents a spacer, e.g. S is -NH-CO-NH-; $-CO-(CH_2)_n-NH-$ where n=1 to 10; -NH-Ph-; $-NH-(CH_2)_n-$, wherein n=1 to 10; -CO-NH-; $-(CH_2)_n-NH-$ where n=1 to 10; $-CO-(CH_2)_n-NH-$, where n=1 to 10; or -CS-NH-;
C represents a metal chelating moiety; and
M represents a lanthanide metal ion coordinated to C.

**13.** A modulator identified by the method of any preceding claim.

**14.** The use of resonance energy transfer, e.g. time resolved resonance energy transfer, to monitor and/or measure a disruption of the molecular interaction between between two binding partners, wherein a first binding partner comprises a luminescent metal complex, the complex optionally including a metal liganding moiety and one or more lanthanide metal ions, and a second binding partner comprises a flurorescent acceptor moiety which is optionally a fluorescent protein, the disruption being caused by a compound that affects binding between the binding partners and which is selected from an enzyme product and an enzyme substrate, said compound being comprised in a product obtained by contacting an enzyme with a substrate for the enzyme in the presence of a potential modulator under conditions effective for the enzymatic activity of the enzyme,
optionally wherein: the resonance energy transfer is measured by exposure to light having a wavelength in the range from 250 nm to 750 nm, the binding partners are as defined in claim 5, the enzyme has an activity defined in claim 8, the fluorescent protein is as defined in claim 10, the lanthanide metal ion is as defined in the optional part of claim 11, and/or the metal complex is as defined in claim 14.

**15.** A composition for use in performing a method for identifying a modulator of an enzymatic activity by monitoring and/or measuring a disruption of the molecular interaction between between two binding partners, the composition comprising
a composition obtainable by contacting the enzyme with a substrate for the enzyme in the presence of a potential modulator under conditions effective for the enzymatic activity of the enzyme;
two binding partners, wherein a first binding partner comprises a luminescent metal complex which optionally includes a metal liganding moiety and one or more lanthanide metal ions, and a second binding partner comprises a fluorescent acceptor moiety which is optionally a fluorescent protein

optionally wherein: the binding partners are as defined in claim 5, the enzyme has an activity defined in claim 8, the fluorescent protein is as defined in claim 10, the lanthanide metal ion is as defined in the optional part of claim 11, and/or the metal complex is as defined in claim 14.

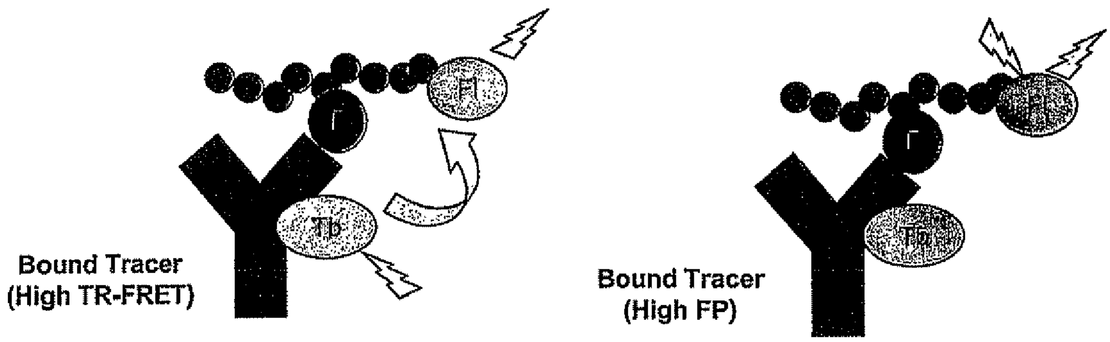


FIG. 1

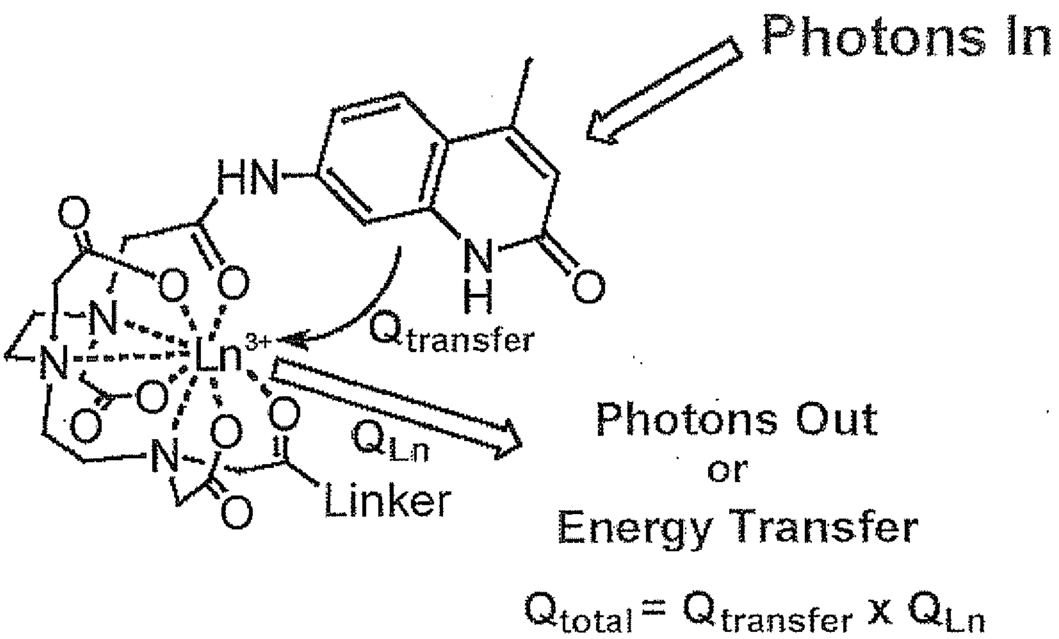
Photons In
Qtransfer
QLn
Linker
Photons Out
or
Energy Transfer
Qtotal = Qtransfer x QLn

FIG. 2

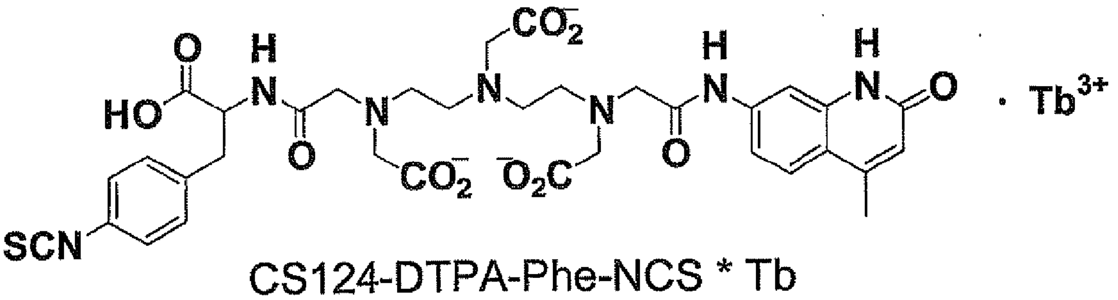

CS124-DTPA-Phe-NCS * Tb

FIG. 3A

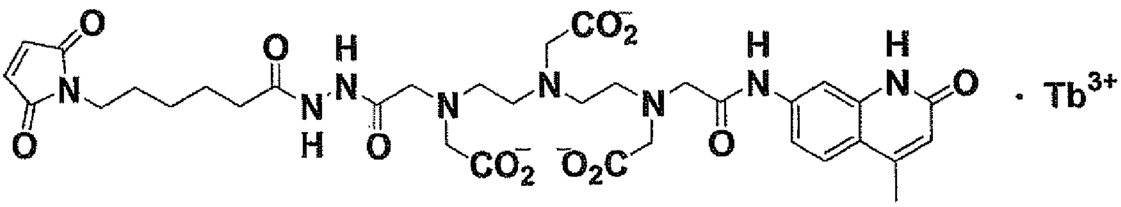

CS124-DTPA-EMCH * Tb

FIG. 3B

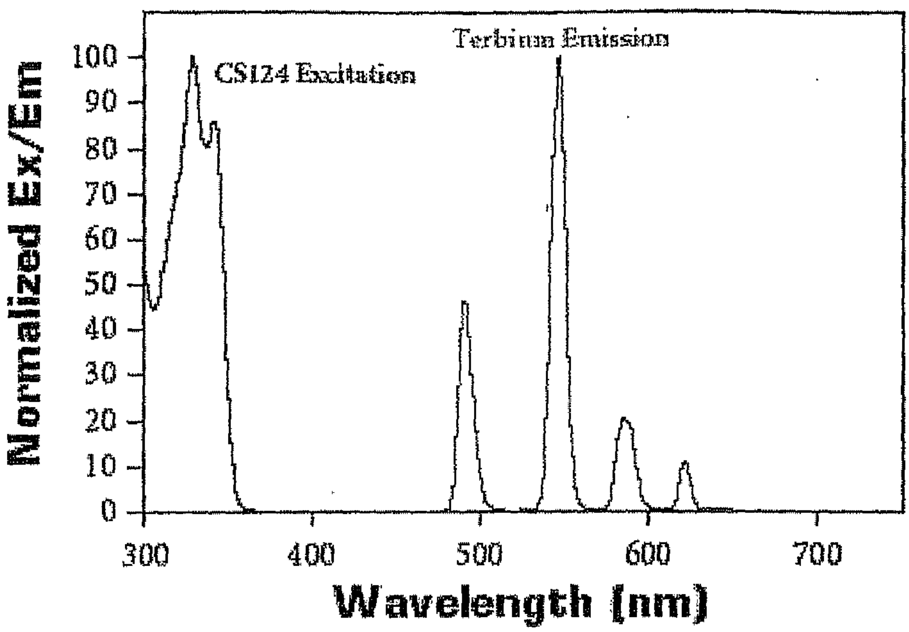
Terbium Emission
CS124 Excitation
Normalized Ex/Em
100
90
80
70
60
50
40
30
20
10
0
300
400
500
600
700
Wavelength (nm)

FIG. 4

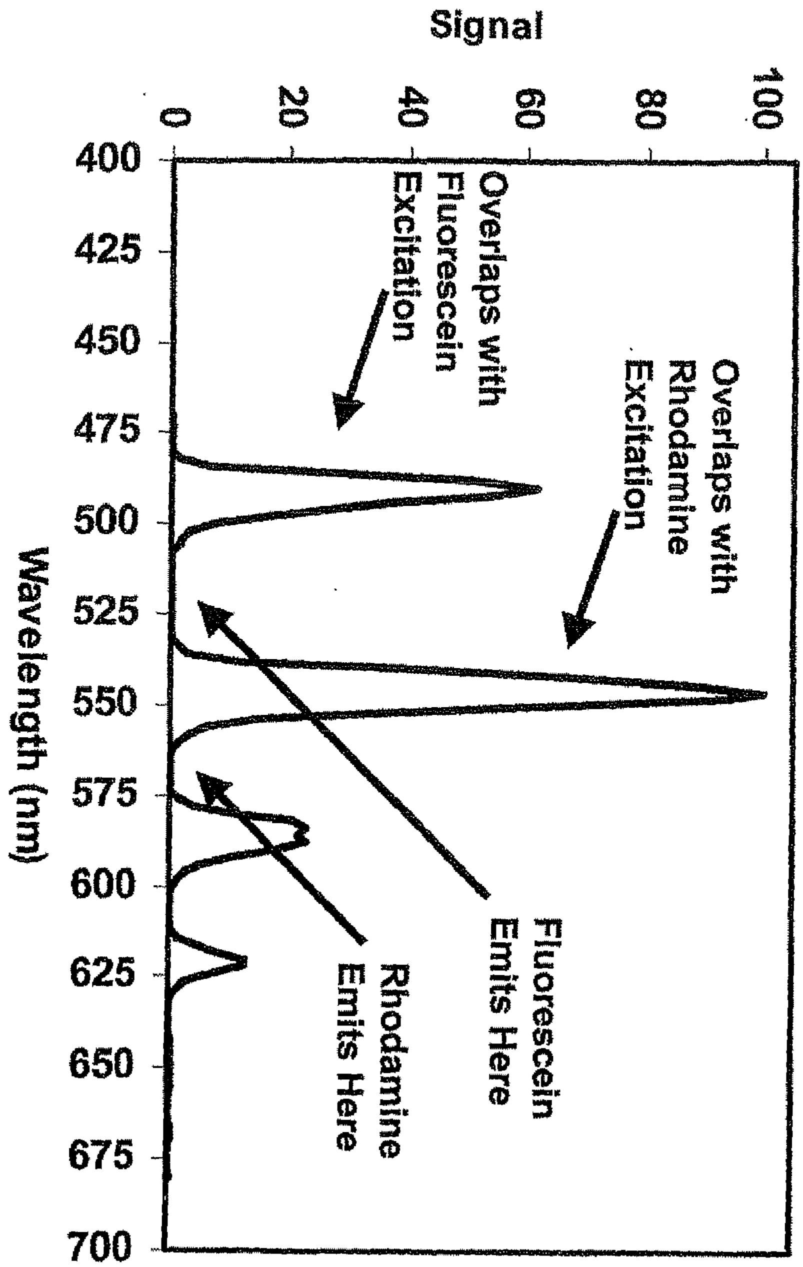
Signal
0
20
40
60
80
100
400
425
450
475
500
525
550
575
600
625
650
675
700
Wavelength (nm)
Overlaps with Fluorescein Excitation
Overlaps with Rhodamine Excitation
Fluorescein Emits Here
Rhodamine Emits Here

FIG. 5

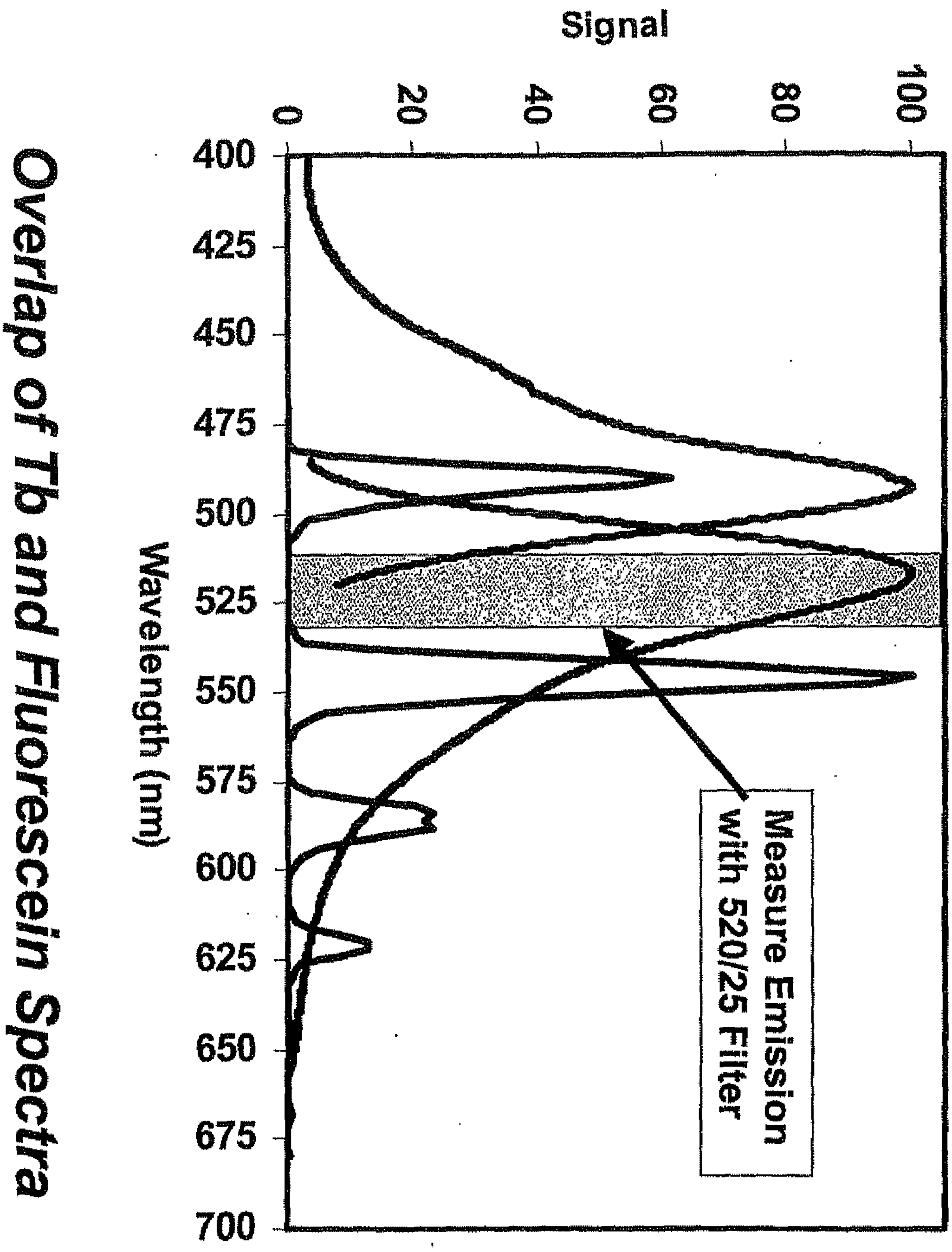
Overlap of Tb and Fluorescein Spectra
Signal
0
20
40
60
80
100
Wavelength (nm)
400
425
450
475
500
525
550
575
600
625
650
675
700
Measure Emission
with 520/25 Filter

FIG. 6

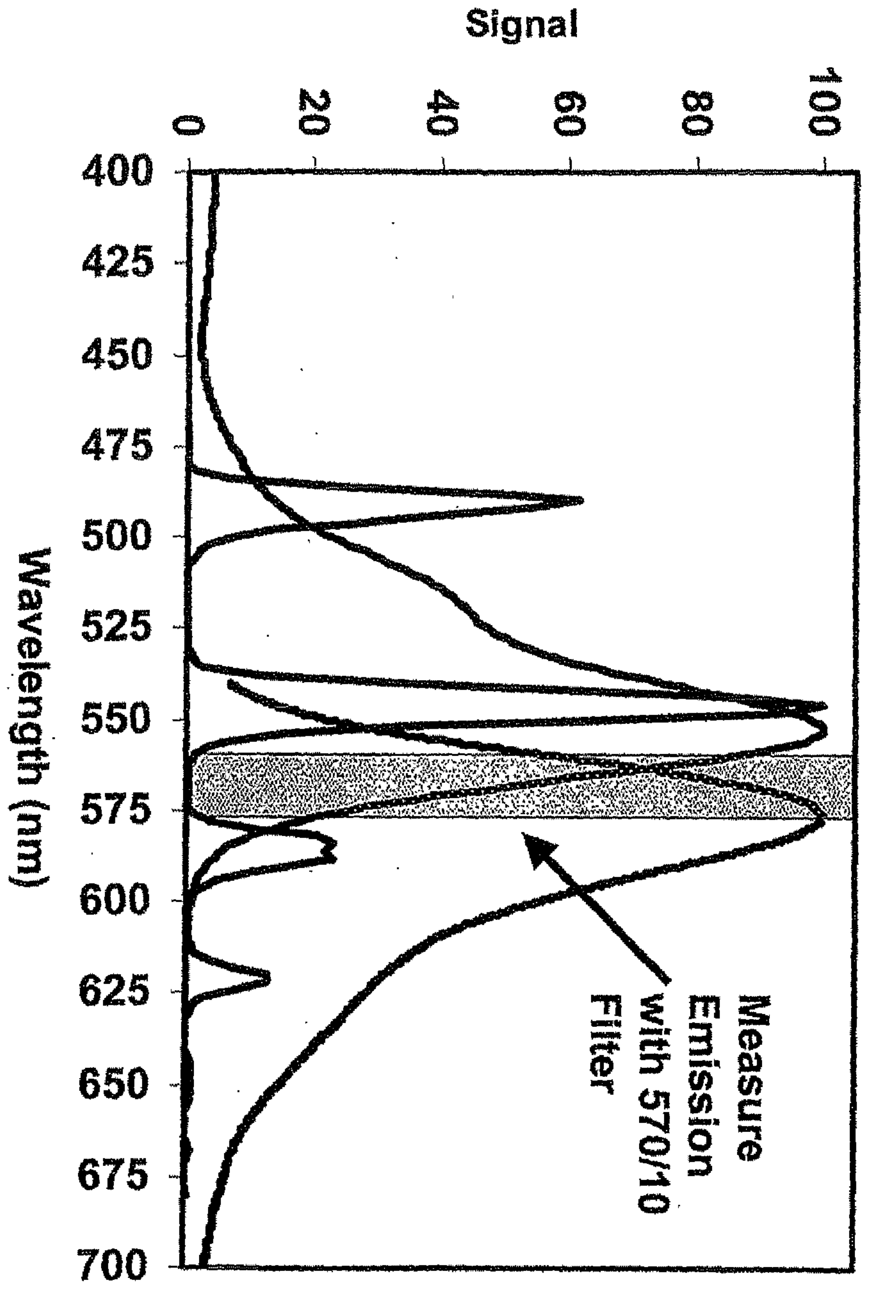
Signal
0
20
40
60
80
100
400
425
450
475
500
525
550
575
600
625
650
675
700
Wavelength (nm)
Measure Emission with 570/10 Filter

FIG. 7

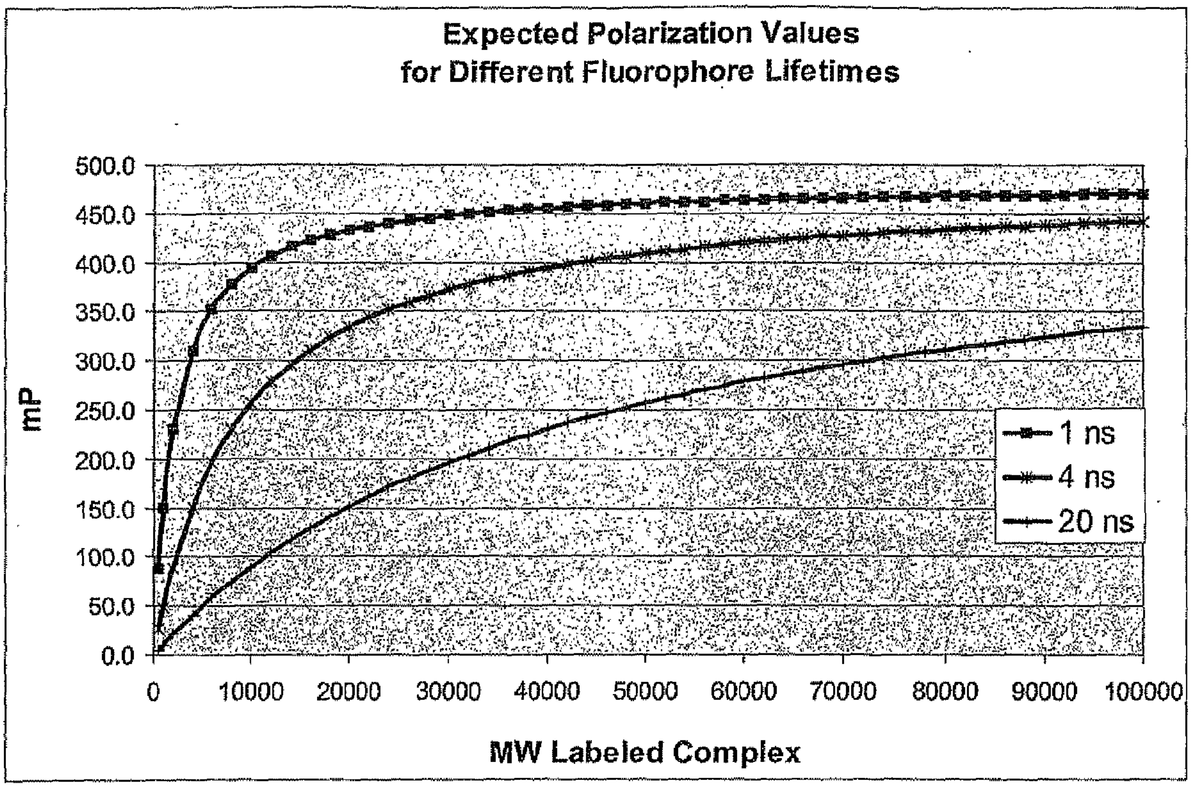
Expected Polarization Values
for Different Fluorophore Lifetimes
mP
500.0
450.0
400.0
350.0
300.0
250.0
200.0
150.0
100.0
50.0
0.0
0
10000
20000
30000
40000
50000
60000
70000
80000
90000
100000
MW Labeled Complex
1 ns
4 ns
20 ns

FIG. 8

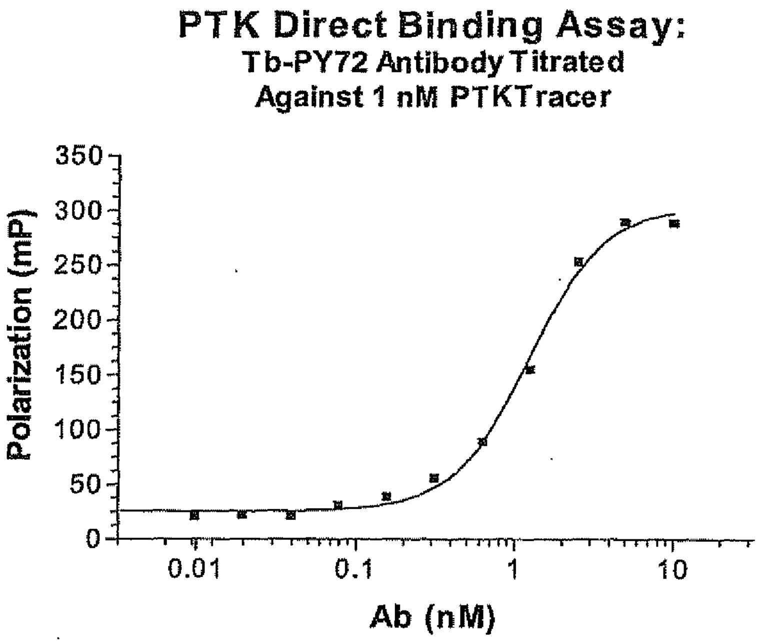
PTK Direct Binding Assay:
Tb-PY72 Antibody Titrated
Against 1 nM PTKTracer
Polarization (mP)
350
300
250
200
150
100
50
0
0.01
0.1
1
10
Ab (nM)

FIG. 9

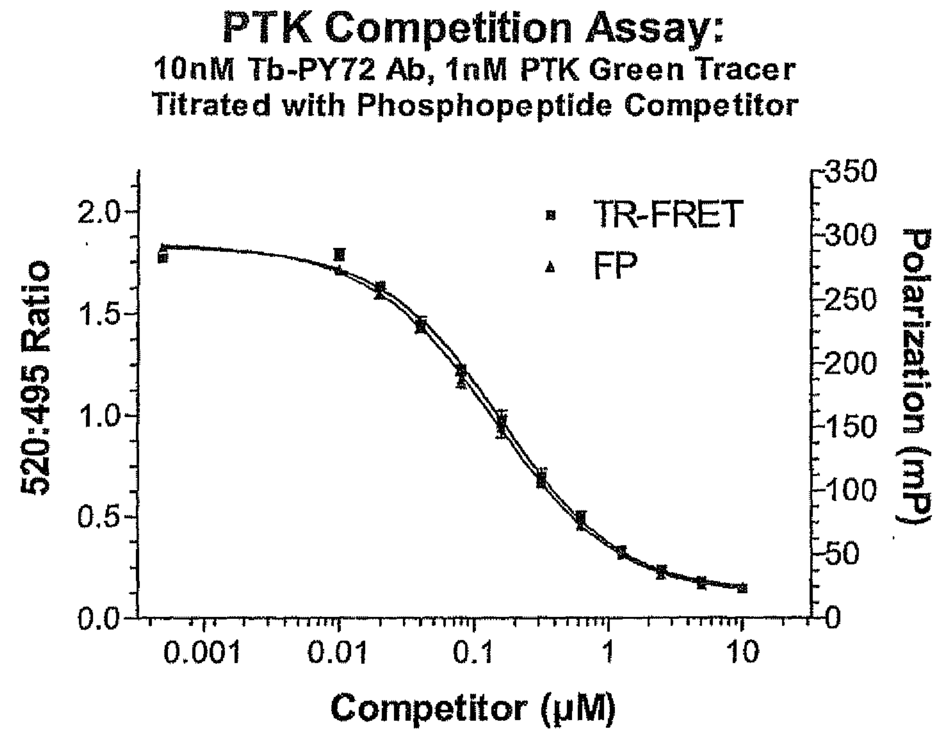
PTK Competition Assay:
10nM Tb-PY72 Ab, 1nM PTK Green Tracer
Titrated with Phosphopeptide Competitor
520:495 Ratio
2.0
1.5
1.0
0.5
0.0
Polarization (mP)
350
300
250
200
150
100
50
0
TR-FRET
FP
0.001
0.01
0.1
1
10
Competitor (μM)

FIG. 10

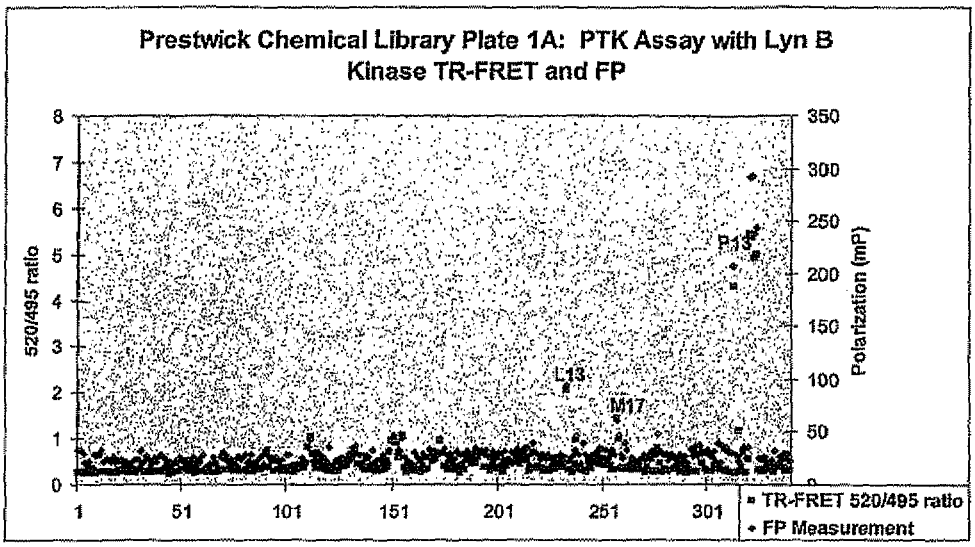


FIG. 11A

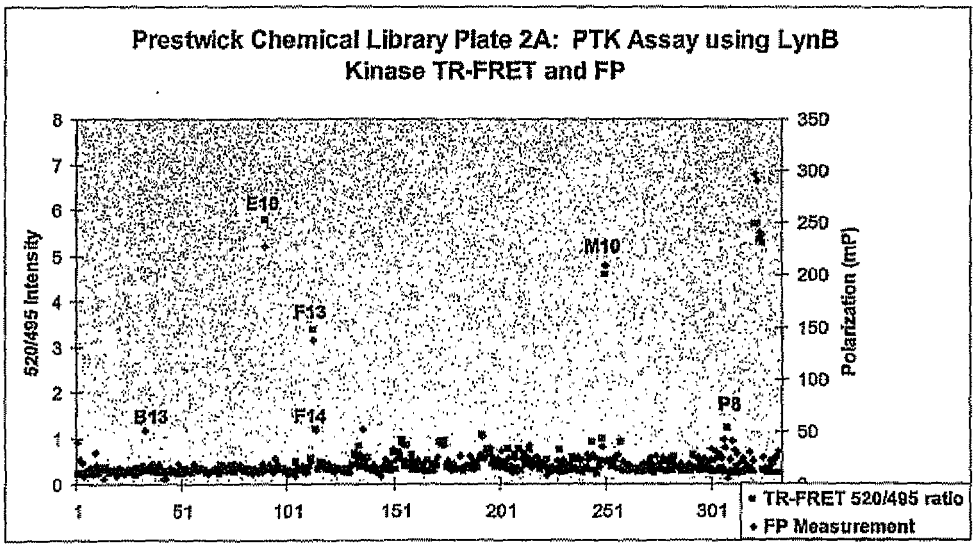


FIG. 11B

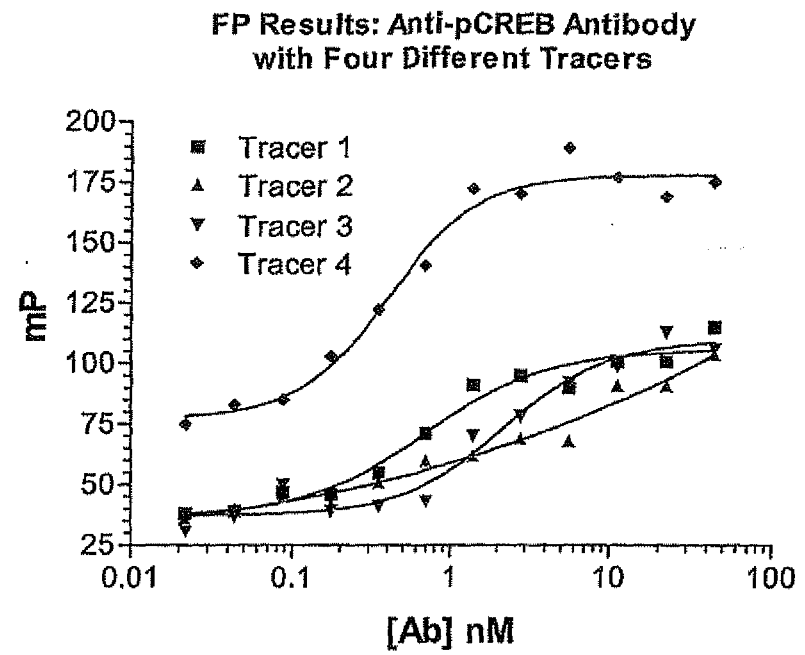


FIG. 12A

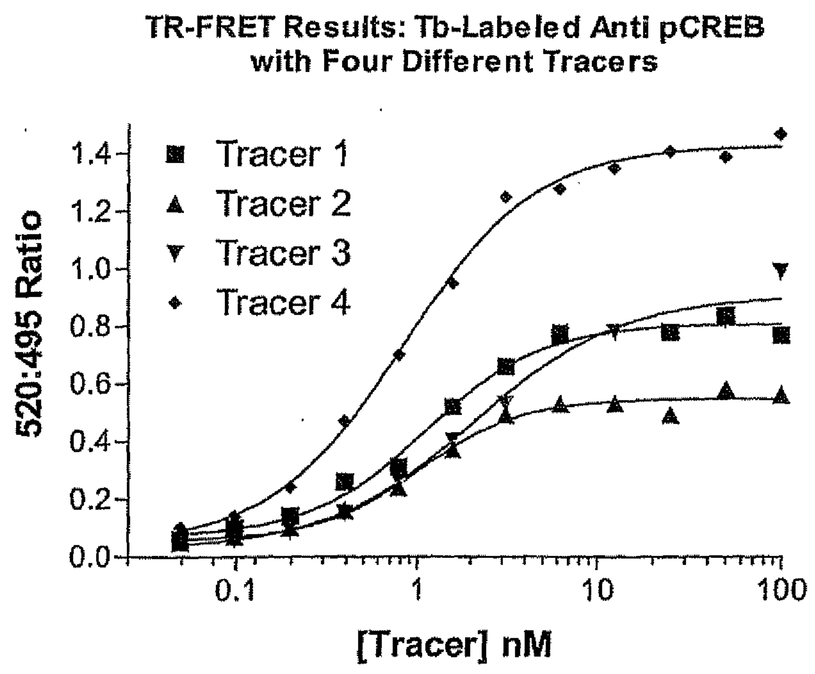


FIG. 12B

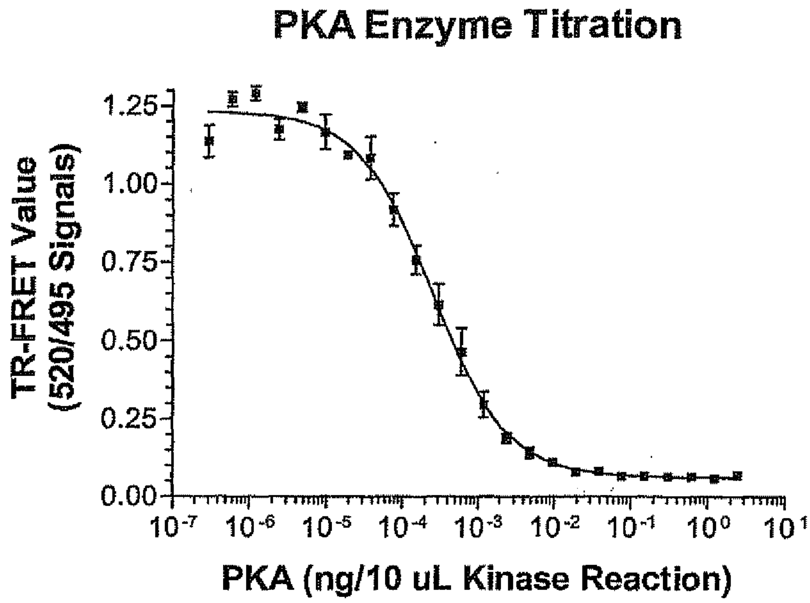


FIG. 13A

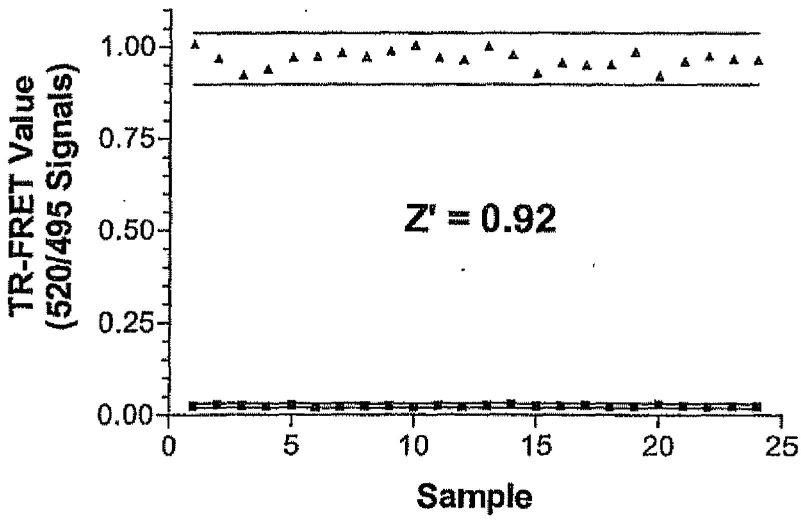


FIG. 13B

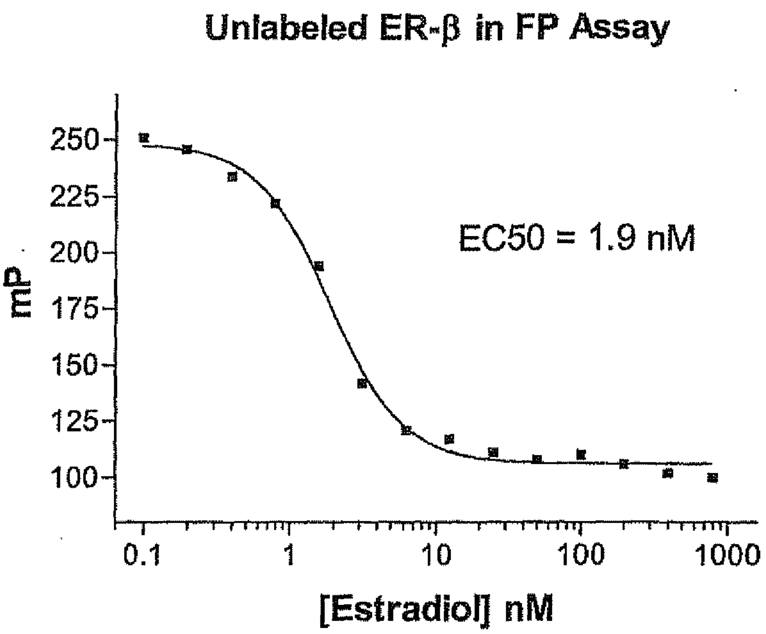


FIG. 14A

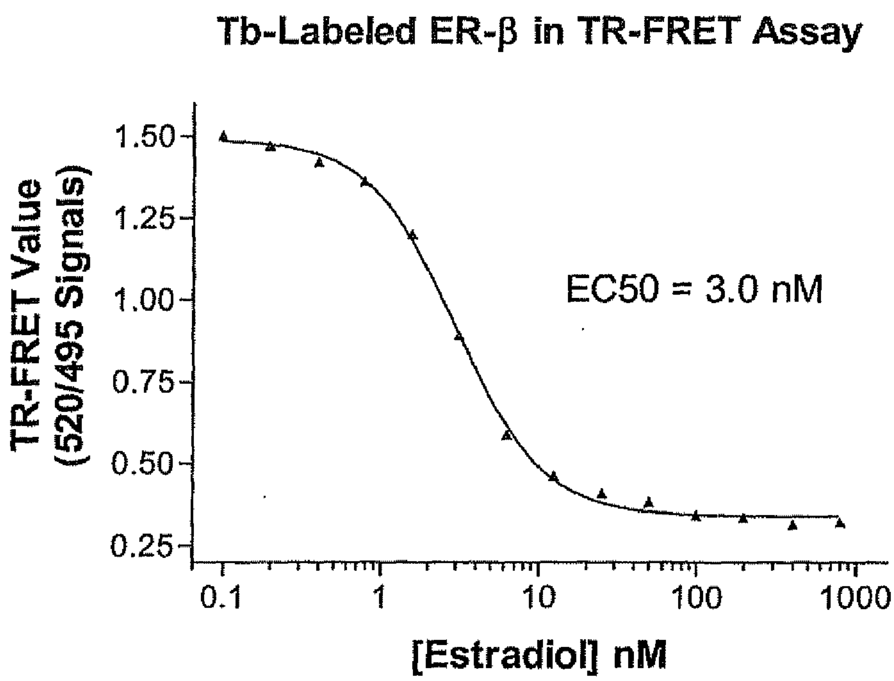


FIG. 14B

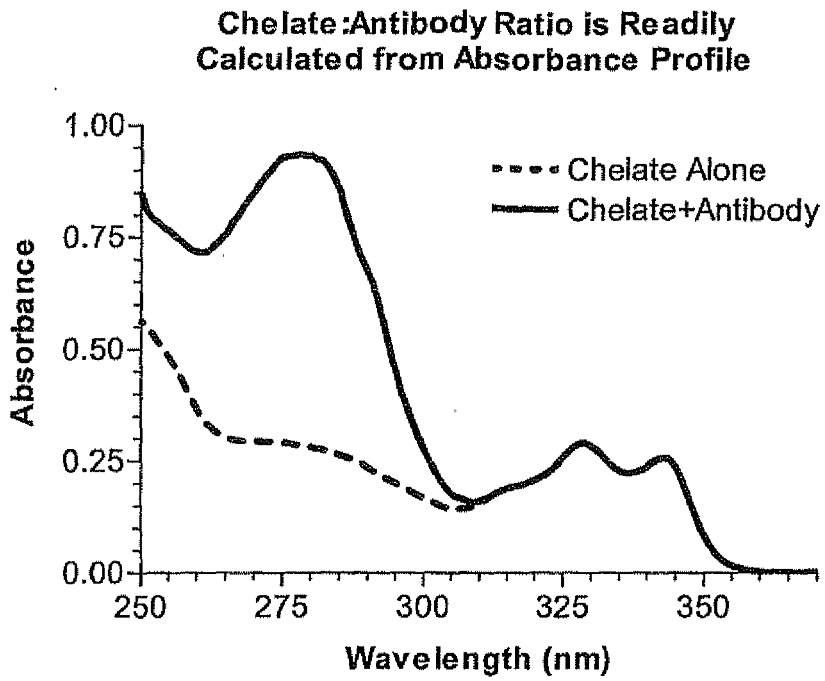
Chelate:Antibody Ratio is Readily
Calculated from Absorbance Profile
1.00
0.75
0.50
0.25
0.00
Absorbance
250
275
300
325
350
Wavelength (nm)
Chelate Alone
Chelate+Antibody

FIG. 15

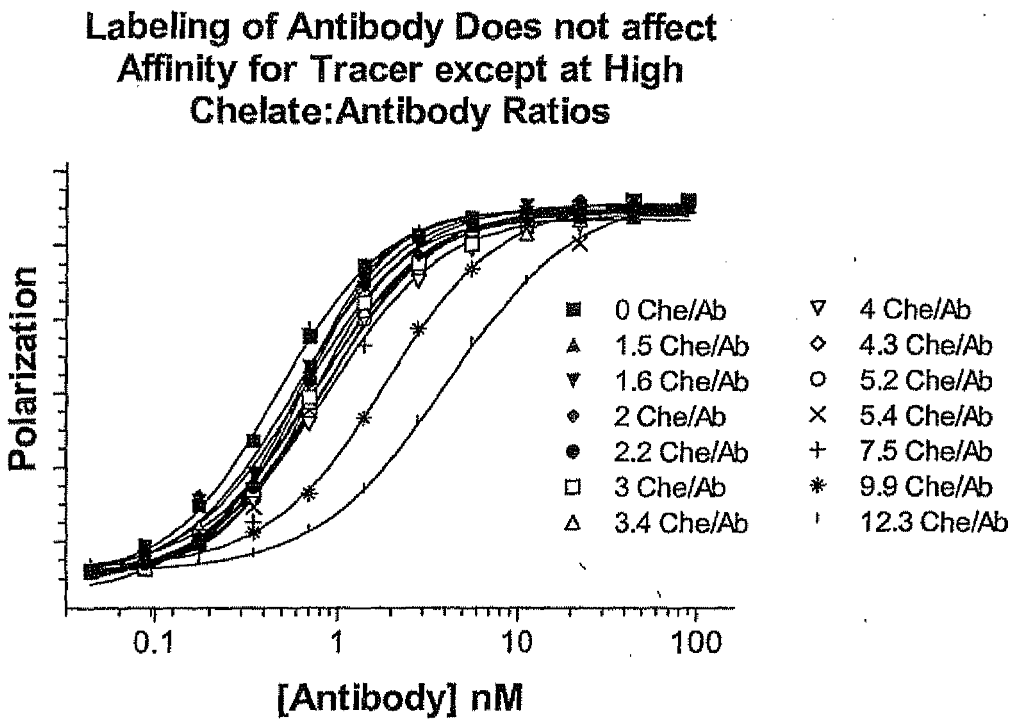
Labeling of Antibody Does not affect Affinity for Tracer except at High Chelate:Antibody Ratios
Polarization
0 Che/Ab
1.5 Che/Ab
1.6 Che/Ab
2 Che/Ab
2.2 Che/Ab
3 Che/Ab
3.4 Che/Ab
4 Che/Ab
4.3 Che/Ab
5.2 Che/Ab
5.4 Che/Ab
7.5 Che/Ab
9.9 Che/Ab
12.3 Che/Ab
0.1
1
10
100
[Antibody] nM

FIG. 16

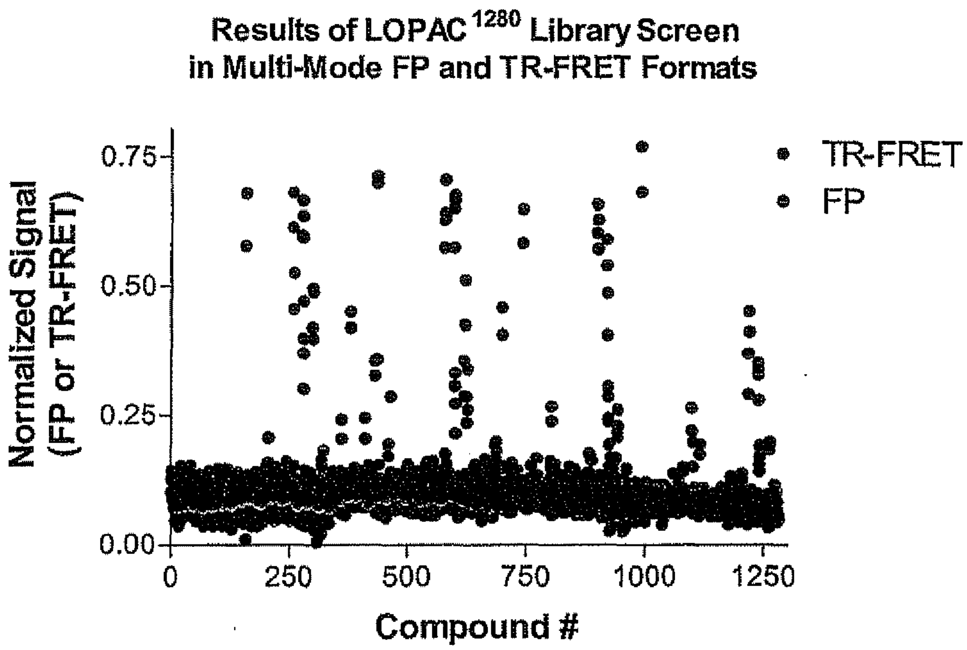
Results of LOPAC 1280 Library Screen
in Multi-Mode FP and TR-FRET Formats
Normalized Signal
(FP or TR-FRET)
0.75
0.50
0.25
0.00
0
250
500
750
1000
1250
Compound #
TR-FRET
FP

FIG. 17

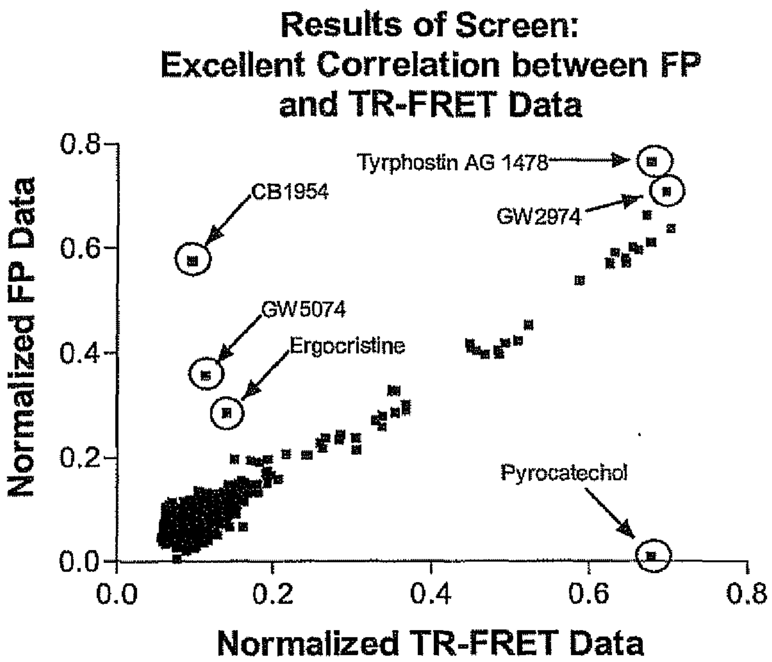
Results of Screen:
Excellent Correlation between FP
and TR-FRET Data
Normalized FP Data
0.8
0.6
0.4
0.2
0.0
Tyrphostin AG 1478
CB1954
GW2974
GW5074
Ergocristine
Pyrocatechol
0.0
0.2
0.4
0.6
0.8
Normalized TR-FRET Data

FIG. 18

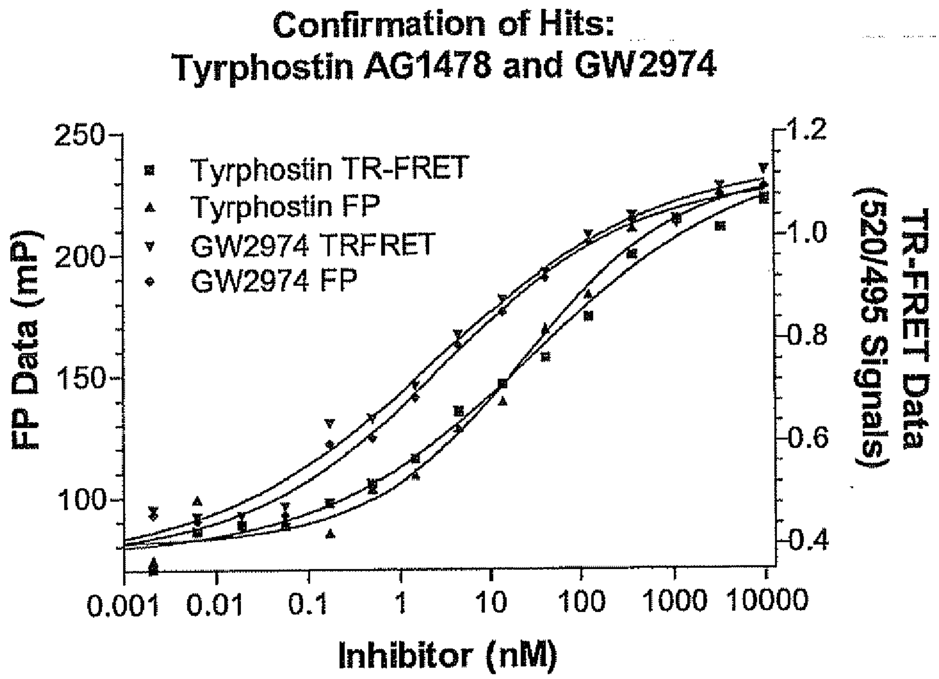
Confirmation of Hits:
Tyrphostin AG1478 and GW2974
FP Data (mP)
250
200
150
100
TR-FRET Data
(520/495 Signals)
1.2
1.0
0.8
0.6
0.4
Tyrphostin TR-FRET
Tyrphostin FP
GW2974 TRFRET
GW2974 FP
0.001
0.01
0.1
1
10
100
1000
10000
Inhibitor (nM)

FIG.19

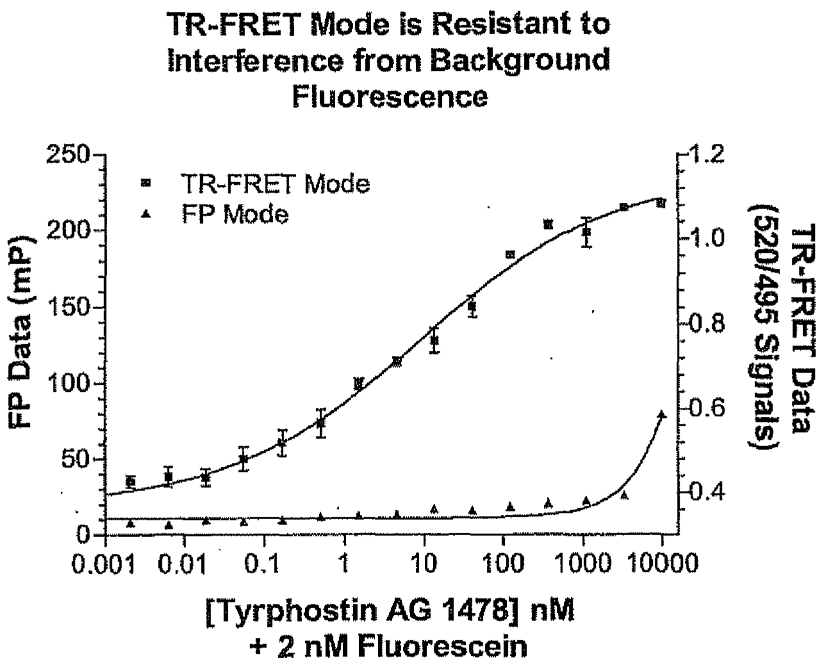
TR-FRET Mode is Resistant to
Interference from Background
Fluorescence
TR-FRET Mode
FP Mode
FP Data (mP)
250
200
150
100
50
0
1.2
1.0
0.8
0.6
0.4
TR-FRET Data
(520/495 Signals)
0.001
0.01
0.1
1
10
100
1000
10000
[Tyrphostin AG 1478] nM
+ 2 nM Fluorescein

FIG. 20

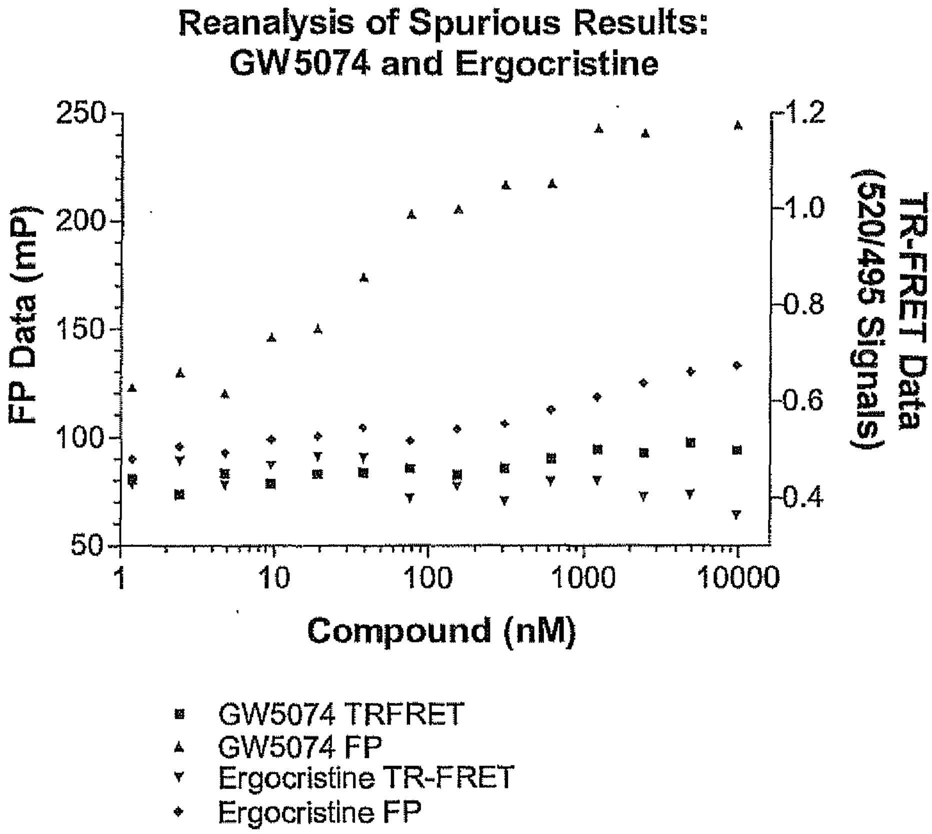
Reanalysis of Spurious Results:
GW5074 and Ergocristine
FP Data (mP)
250
200
150
100
50
TR-FRET Data
(520/495 Signals)
1.2
1.0
0.8
0.6
0.4
1
10
100
1000
10000
Compound (nM)
GW5074 TRFRET
GW5074 FP
Ergocristine TR-FRET
Ergocristine FP

FIG. 21

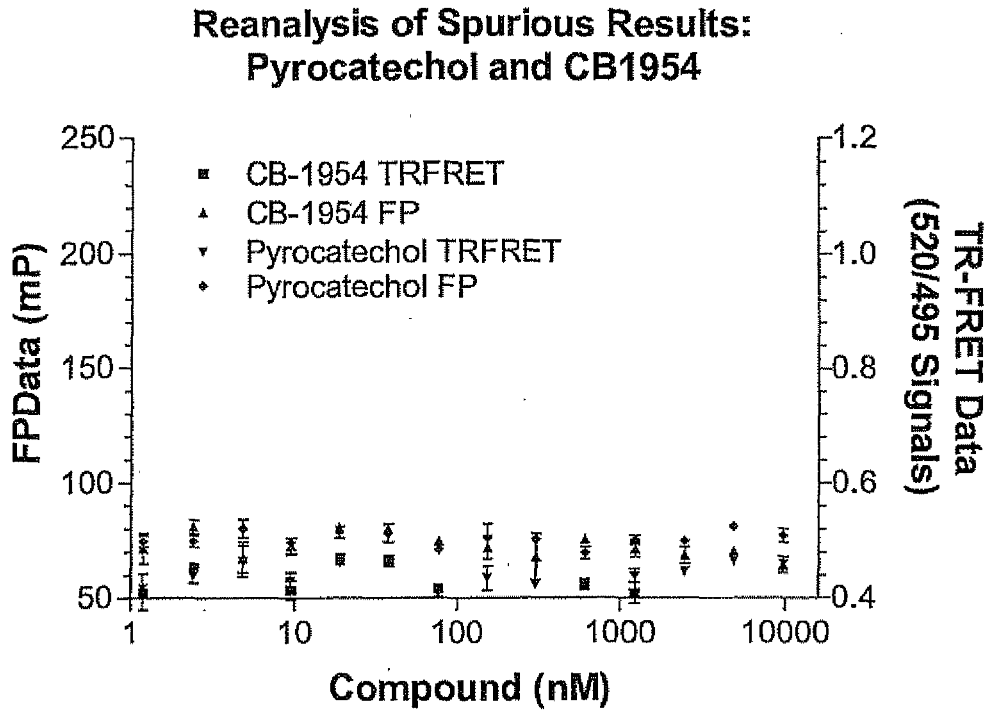
Reanalysis of Spurious Results:
Pyrocatechol and CB1954
250
200
150
100
50
1.2
1.0
0.8
0.6
0.4
1
10
100
1000
10000
CB-1954 TRFRET
CB-1954 FP
Pyrocatechol TRFRET
Pyrocatechol FP
FPData (mP)
TR-FRET Data
(520/495 Signals)
Compound (nM)

FIG. 22

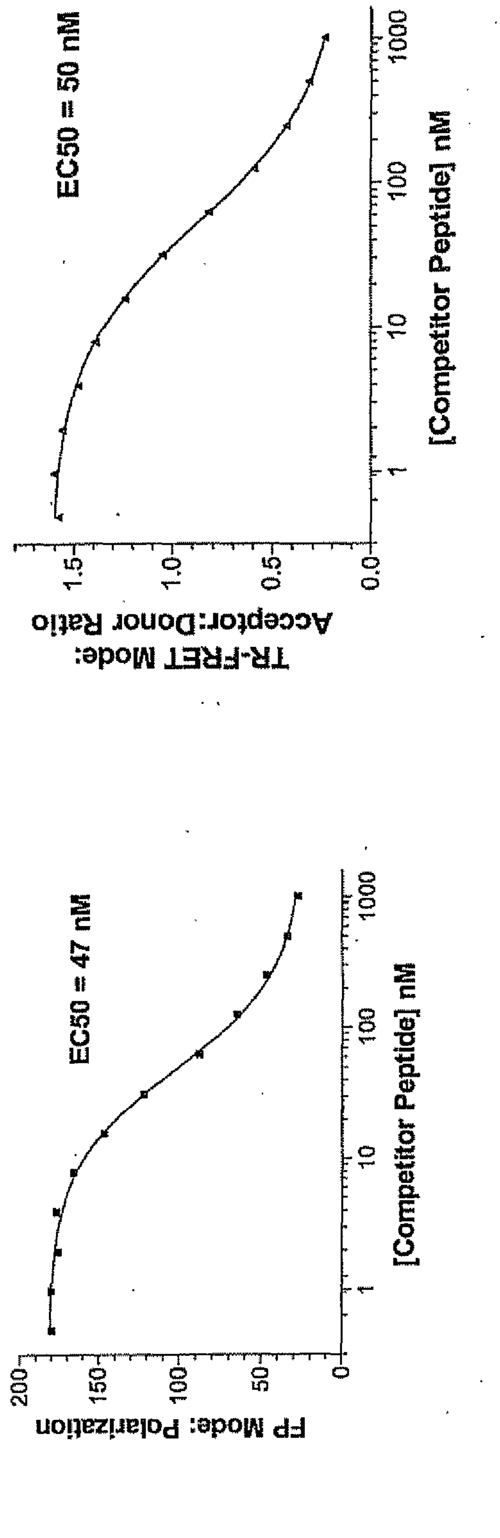
EC50 = 50 nM
TR-FRET Mode:
Acceptor:Donor Ratio
[Competitor Peptide] nM
EC50 = 47 nM
FP Mode: Polarization
[Competitor Peptide] nM

FIG. 23

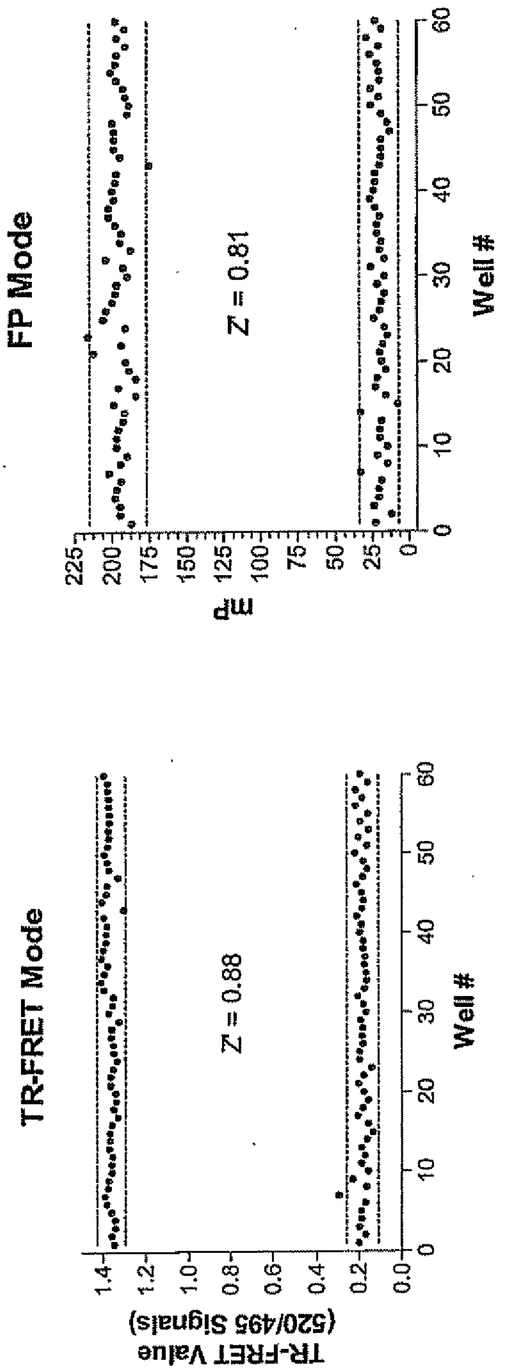
TR-FRET Mode
Z' = 0.88
TR-FRET Value
(520/495 Signals)
Well #
FP Mode
Z' = 0.81
mP
Well #

FIG. 24

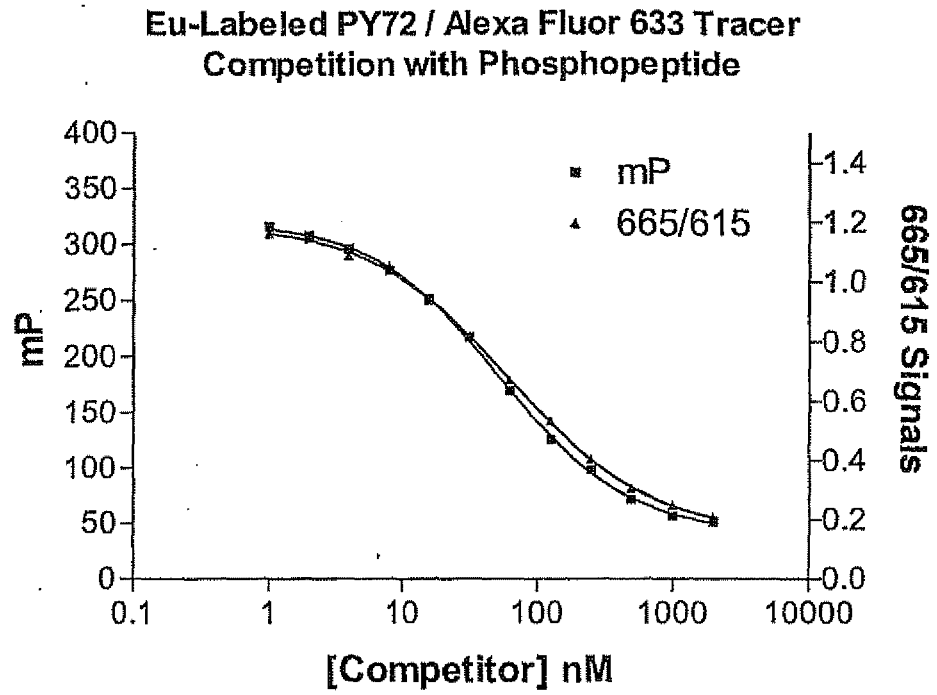
Eu-Labeled PY72 / Alexa Fluor 633 Tracer
Competition with Phosphopeptide
mP
665/615
400
350
300
250
200
150
100
50
0
1.4
1.2
1.0
0.8
0.6
0.4
0.2
0.0
665/615 Signals
0.1
1
10
100
1000
10000
[Competitor] nM

FIG. 25

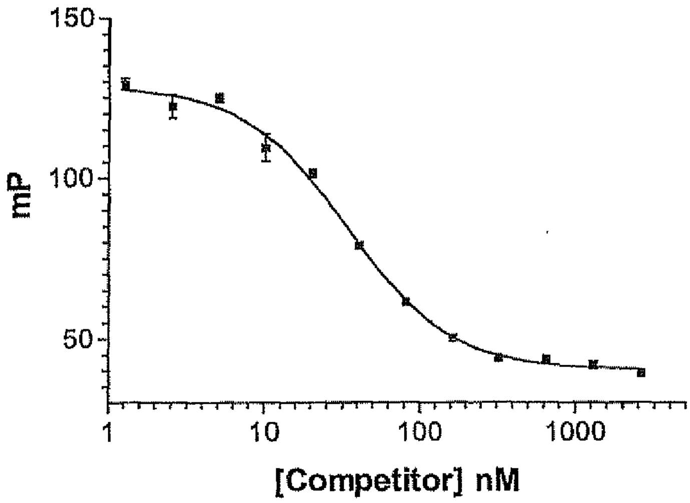


FIG. 26A

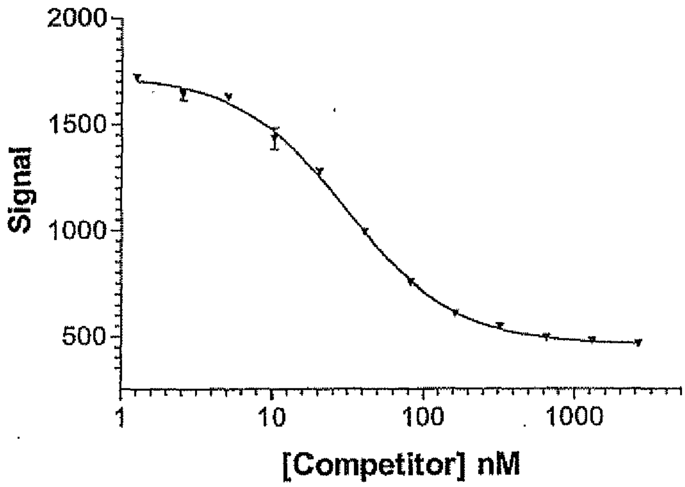


FIG. 26B

Europäisches Patentamt
European Patent Office
Office européen des brevets

# EUROPEAN SEARCH REPORT

Application Number
EP 10 15 2972

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/75167 A2 (LJL BIOSYSTEMS INC; SPORTSMAN J R; HOEKSTRA M F) 14 December 2000 (2000-12-14) * the whole document, in particular page 16, lines 16-29; page 17, lines 9-11, 27-29; page 18, line 22 to page 19, line 4; page 20, line 23 to page 21, line 2; page 24, lines 3-10; page 33, lines 17-25; page 42, lines 6-16; claims 32-40, 42 * ----- | 1-15 | INV. G01N33/58 G01N33/542 G01N33/573 |
| X | UMEZAWA Y ET AL: "Methods of analysis for chemicals that promote/disrupt cellular signaling." ANAL SCI, vol. 18, no. 5, May 2002 (2002-05), pages 503-516, XP002592169 ISSN: 0910-6340 * the whole document, in particular title; abstract; section 3.1.3; figure 4 * ----- | 1-15 | |
| X | GRAY A ET AL: "Nonradioactive methods for the assay of phosphoinositide 3-kinases and phosphoinositide phosphatases and selective detection of signaling lipids in cell and tissue extracts." ANAL BIOCHEM, vol. 313, no. 2, 15 February 2003 (2003-02-15), pages 234-245, XP002592170 * the whole document, in particular title; abstract; page 236, column 1, lines 26-43; figure 1B; page 244, column 2, lines 1-5 * ----- | 1-15 | TECHNICAL FIELDS SEARCHED (IPC) G01N |
| A | SELVIN P R: "PRINCIPLES AND BIOPHYSICAL APPLICATIONS OF LANTHANIDE-BASED PROBES" ANNU REV BIOPH BIOM, vol. 31, 1 January 2002 (2002-01-01), pages 275-302, XP008060956 * the whole document * ----- | 1-15 | |

The present search report has been drawn up for all claims

1

| Place of search | Date of completion of the search | Examiner |
|---|---|---|
| Munich | 16 July 2010 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 15 2972

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2010

| Patent document cited in search report | Publication date | Patent family member(s) | Publication date |
|---|---|---|---|
| WO 0075167 A2 | 14-12-2000 | EP 1261621 A2 | 04-12-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 50237703 P
- US 4945778 A
- WO 0301115 A
- WO 6410255 A
- WO 9623526 A
- WO 0109188 A
- WO 0108712 A
- WO 03011115 A
- US 5639615 A
- US 5656433 A
- US 5622821 A
- US 5571897 A
- US 5534622 A
- US 5220012 A
- US 5162508 A
- US 4927923 A
- US 5221623 A, Thompson
- US 5683888 A, Campbell
- US 5674713 A, DeLuca
- US 5650289 A, Wood
- US 5843746 A, Tatsumi
- US 6410255 B
- US 5625048 A, Tsien
- US 5777079 A, Tsien
- US 5804387 A, Cormack
- US 6511815 B
- US 5445935 B
- US 4822733 A
- US 5527684 A
- US 6352672 B


### Non-patent literature cited in the description


- **Lakowicz, J.R.** Topics in Fluorescence Spectroscopy. Plenum Press, 1991
- Emerging applications of florescence spectroscopy to cellular imaging: lifetime imaging, metal-ligand probes, multi photon excitation and light quenching. **Lakowicz, J. R.** Scanning Microsc. 1996, vol. 10, 213-24
- **Sambrook et al.** Molecular Cloning: A Laboratory Manual. Cold Spring Harbor Laboratory Press, 1989
- Cells: A Laboratory Manual. Cold Spring Harbor Laboratory Press, 1998
- **Snyder ; Love.** Optical Waveguide Theory. Chapman & Hall
- **Lakowicz, J.R.** Topics in Fluorescence Spectroscopy. Plenum Press, 1991, vol. 1-3
- Resonance Energy Transfer Microscopy. **Herman, B.** Fluorescence Microscopy of Living Cells in Culture, Part B, Methods in Cell Biology. Academic Press, 1989, vol. 30, 219-243
- **Turro, N.J.** Modem Molecular Photochemistry. Menlo Park: Benjamin/Cummings Publishing Col, Inc, 1978, 296-361
- **Bernard Valeur.** Molecular Fluorescence: Principles and Applications. Wiley VCH, 2002
- **Berlman, I.B.** Energy transfer parameters of aromatic compounds. Academic Press, 1973
- Molecular Probes Catalog. 2003
- **Tsien et al.** Handbook of Biological Confocal Microscopy. 1990, 169-178
- The American Chemical Society Style Guide. American Chemical Society, 1997
- 2001 Guidelines for Authors. *J. Org. Chem.,* vol. 66 (1), 24A
- A Short Guide to Abbreviations and Their Use in Peptide Science. *J. Peptide. Sci.,* 1999, vol. 5, 465-471
- **Spatola, A.F.** Chemistry and Biochemistry of Amino Acids, Peptides and Proteins. Marcel Decker, 1983, 267
- **Kohler et al.** *Nature,* 1975, vol. 256, 495-497
- **Kosbor et al.** *Immunology Today,* 1983, vol. 4, 72
- **Cote et al.** *Proc. Natl. Acad. Sci. USA,* 1983, vol. 80, 2026-2030
- **Cole et al.** Monoclonal Antibodies and Cancer Therapy. Alan R. Liss, Inc, 1983, 77-96
- **Huse et al.** *Science,* 1989, vol. 246, 1275-1281
- Short Protocols in Molecular Biology. Green Publishing Associates and John Wiley & Sons, 1992
- **Flohe et al.** *Biochim. Biophys. Acta.,* 1970, vol. 220, 469-476
- **Tilgmann et al.** *FEBS,* 1990, vol. 264, 95-99
- PCR Primer: A Laboratory Manual. Cold Spring Harbor Laboratory Press, 1995
- **Lewis.** *Genetic Engineering News,* 1992, vol. 12 (9), 1

- **Guatelli et al.** *Proc. Natl. Acad. Sci. USA,* 1990, vol. 87, 1874-1878
- **Weiss.** *Science,* 1991, vol. 254, 1292
- **Green.** Fluorescent Proteins. Academic Press, 19-47
- Polarization of the fluorescence of solutions. **Weber.** Fluorescence and Phosphorescence Analysis. Interscience Publishers, 1966, 217-240
- **Zhang et al.** A Simple Statistical Parameter for Use in Evaluation and Validation of High Throughput Screening Assays. *Journal of Biomolecular Screening,* 1999, vol. 4 (2), 67-73

SEQUENCE LISTING

<110> Life Technologies Corporation

<120> Multiplex Binding and Activity Assays

<130> CHW/P115954EP2

<150> US60/502377
<151> 2003-09-12

<160> 12

<170> PatentIn version 3.5

<210> 1
<211> 11
<212> PRT
<213> Artificial Sequence

<220>
<223> Synthetically generated polypeptide

<220>
<221> MOD_RES
<222> (1)..(1)
<223> Xaa = fluorescein

<220>
<221> MOD_RES
<222> (5)..(5)
<223> Xaa = phosphorylated tyrosine

<400> 1

```
Xaa Ala Asp Glu Xaa Pro Ile Pro Gln Gln Ser
1               5                   10
```

<210> 2
<211> 12
<212> PRT
<213> Artificial Sequence

<220>
<223> Synthetically generated polypeptide

<220>
<221> MOD_RES
<222> (1)..(1)
<223> Xaa = fluorescein

<220>
<221> MOD_RES
<222> (8)..(8)
<223> Xaa = phosphorylated serine

<400> 2

```
Xaa Gly Arg Pro Arg Thr Ser Xaa Phe Ala Glu Gly
1               5                   10
```

<210> 3
<211> 10
<212> PRT
<213> Artificial Sequence

<220>
<223> Synthetically generated polypeptide

<220>
<221> MOD_RES
<222> (4)..(4)
<223> Xaa = phosphorylated tyrosine

<400> 3

Ala Asp Glu Xaa Leu Ile Pro Gln Gln Ser
1 5 10

<210> 4
<211> 15
<212> PRT
<213> Artificial Sequence

<220>
<223> Synthetically generated polypeptide

<220>
<221> MOD_RES
<222> (1)..(1)
<223> Xaa = fluorescein

<220>
<221> MOD_RES
<222> (12)..(12)
<223> Xaa = phosphorylated serine

<400> 4

Xaa Leu Arg Arg Glu Ile Leu Ser Arg Arg Pro Xaa Tyr Arg Lys
1 5 10 15

<210> 5
<211> 13
<212> PRT
<213> Artificial Sequence

<220>
<223> Synthetically generated polypeptide

<220>
<221> MOD_RES
<222> (1)..(1)
<223> Xaa = fluorescein

<220>
<221> MOD_RES
<222> (10)..(10)
<223> Xaa = phosphorylated serine

<400> 5

Xaa Arg Glu Ile Leu Ser Arg Arg Pro Xaa Tyr Arg Lys
1 5 10

<210> 6
<211> 11
<212> PRT

<213> Artificial Sequence

<220>
<223> Synthetically generated polypeptide

<220>
<221> MOD_RES
<222> (1)..(1)
<223> Xaa = fluorescein

<220>
<221> MOD_RES
<222> (8)..(8)
<223> Xaa = phosphorylated serine

<400> 6

```
Xaa Ile Leu Ser Arg Arg Pro Xaa Tyr Arg Lys
1               5                   10
```

<210> 7
<211> 15
<212> PRT
<213> Artificial Sequence

<220>
<223> Synthetically generated polypeptide

<220>
<221> MOD_RES
<222> (11)..(11)
<223> Xaa = phosphorylated serine

<220>
<221> MOD_RES
<222> (15)..(15)
<223> Xaa = fluorescein

<400> 7

```
Leu Arg Arg Glu Ile Leu Ser Arg Arg Pro Xaa Tyr Arg Lys Xaa
1               5                   10                  15
```

<210> 8
<211> 14
<212> PRT
<213> Artificial Sequence

<220>
<223> Synthetically generated polypeptide

<400> 8

```
Leu Arg Arg Glu Ile Leu Ser Arg Arg Pro Ser Tyr Arg Lys
1               5                   10
```

<210> 9
<211> 11
<212> PRT
<213> Artificial Sequence

<220>
<223> Synthetically generated polypeptide

<220>
<221> MOD_RES
<222> (5)..(5)
<223> Xaa = phosphorylated tyrosine

<400> 9

Cys Ala Asp Glu Xaa Leu Ile Pro Gln Gln Ser
1 5 10

<210> 10
<211> 12
<212> PRT
<213> Artificial Sequence

<220>
<223> Synthetically generated polypeptide

<220>
<221> MOD_RES
<222> (1)..(1)
<223> Xaa = Alexa Fluor 633

<220>
<221> MOD_RES
<222> (6)..(6)
<223> Xaa = phosphorylated tyrosine

<400> 10

Xaa Cys Ala Asp Glu Xaa Leu Ile Pro Gln Gln Ser
1 5 10

<210> 11
<211> 7
<212> PRT
<213> Artificial Sequence

<220>
<223> Synthetically generated polypeptide

<220>
<221> MOD_RES
<222> (1)..(1)
<223> Xaa = fluorescein

<400> 11

Xaa His His His His His His
1 5

<210> 12
<211> 10
<212> PRT
<213> Artificial Sequence

<220>
<223> Synthetically generated polypeptide

<220>
<221> MOD_RES
<222> (1)..(1)

<223> Xaa = biotin

<400> 12

```
Xaa Lys Gly Gly His His His His His His
1               5                   10
```